# EUROPEAN PATENT SPECIFICATION

(11) **EP 0 842 933 B1**
(45) Date of publication and mention of the grant of the patent: **04.08.2004**
(21) Application number: 96914458.3
(22) Date of filing: 28.05.1996
(51) Int. Cl.: C07D 471/04, A61K 31/435

(54) **NOVEL NAPHTHYRIDINE DERIVATIVES**
NEUE NAPHTHYRIDINDERIVATE.
NOUVEAUX DERIVES DE NAPHTYRIDINE

(30) Priority: 31.05.1995 JP 15847595
(43) Date of publication of application: 20.05.1998
(73) Proprietor: SUMITOMO PHARMACEUTICALS COMPANY, LIMITED, Osaka-shi, Osaka 541-8510 (JP)
(72) Inventor: MURAOKA, Masami, Osaka 565-0811 (JP); IORIYA, Katsuhisa, Osaka 560-0083 (JP); OHASHI, Naohito, Osaka 569-1020 (JP)
(74) Representative: VOSSIUS & PARTNER
(86) International application number: PCT/JP1996/001429
(87) International publication number: WO 1996/038445

(56) References cited:
- EP-A- 0 354 994
- JP-A- 3 181 465
- JP-A- 3 223 254
- JP-A- 6 501 025
- HIROYUKI TAWADA, MYLES HARCOURT, NORIAKI KAWAMURA ET AL. : "Novel Acyl-CoA:Cholesterol Acyltransferase Inhibitors. Synthesis and Biological Activity of 3-Quinolylurea derivatives" J.MED.CHEM., vol. 37, 1994, pages 2079-2084, XP002159316

## Description

### TECHNICAL FIELD

The present invention relates to a naphthyridine derivative or an acid addition salt thereof, which shows acyl-CoA: cholesterol acyl transferase (ACAT) inhibitory activity, and is useful as an agent for treatment of hyperlipidemia and atherosclerosis, and a use thereof.

### BACKGROUND ART

Cerebral vessel disorders such as stroke, or myocardial infarction, which rank in high in causes of death in developed countries, break out with being accompanied by atherosclerosis as basal disease. From the results of epidemiology research, it is pointed out that hypercholesterolemia is one of risk factors for atherosclerosis, and there are mainly used anti-hyperlipidemic agents which can reduce cholesterol level in blood in the prophylaxis or treatment. However, there is no sufficiently effective agent. Recently, it is observed that cells derived from macrophage accumulate cholesterol ester droplets within the cells and become foam cells in atherosclerotic lesions, and it is clarified that these foam cells deeply participate in the developments of atherosclerotic lesions (Atherosclerosis, **10**, 164-177, 1990). In addition, it is reported that ACAT activity is increased and cholesterol esters are accumulated in the vasucular wall of atherosclerotic lesions (Biochem. Biophys. Acta, **617**, 458-471, 1980). Therefore, an inhibitor of ACAT, which catalyses cholesterol esterification, is expected to suppress the formation or the development of atheroschlerotic lesions as a result of the inhibition of foam cell formation and of cholesterol accumulation in vascular wall.

On the other hand, cholesterol in food is absorbed in the free form at intestinal epidermal cells, and then released in the form of chylomicron esterified by ACAT into the blood. Therefore, an inhibitor of ACAT is expected to reduce the cholesterol level in the blood caused by the inhibition of absorption of cholesterol in food at the intestine and reabsorption of cholesterol released into the intestine (J. Lipid. Research, **34**, 279-294, 1993).

Japanese Patent Publication (Kokai) Nos. 3-181465, 3-223254 and Japanese Patent Publication (Kohyo) No. 6-501025 disclose some kinds of quinoline derivatives having ACAT inhibitory activity, and Japanese Patent Publication (Kokai) No. 5-32666 discloses some kinds of thienopyridine derivatives having an ACAT inhibitory activity, but these compounds are different in structure from the present compounds.

EP A 0 354 994 deals with quinoline derivatives active as ACAT inhibitors. In addition in J. Med. Chem., vol.37, 1997, pages 2079-2084 it is reported that 3-Quinolylurea derivatives have ACAT inhibitory activity.

### DISCLOSURE OF INVENTION

An object of the present invention is to provide a naphthyridine derivative having an ACAT inhibitory activity and being useful as an agent for treatment of hyperlipidemia and atherosclerosis.

The present inventors have intensively studied in order to obtain a novel compound having a potent ACAT inhibitory activity, and have found that a novel naphthyridine derivative of the following formula (1) and an acid addition salt thereof show such an activity, and then have accomplished the present invention.

A naphthyridine derivative of the formula: 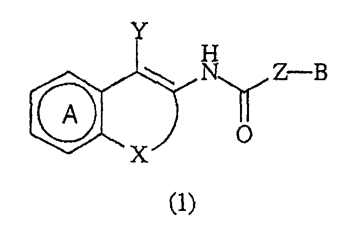 wherein Ring A is a substituted or unsubstituted pyridine ring,
X is a group of the formula: 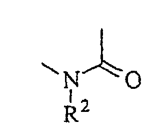 wherein R² is an alkyl group, a substituted alkyl group, an alkenyl group, a substituted alkenyl group,
Z is -NH-,
Y is , a , phenyl group or a substituted phenyl group,
B is an aromatic group, a substituted aromatic group, or an acid addition salt thereof.

Each group in the present invention is explained below.

Ring A is a substituted or unsubstituted pyridine ring, selected from the following formulae (a), (b) and (c).

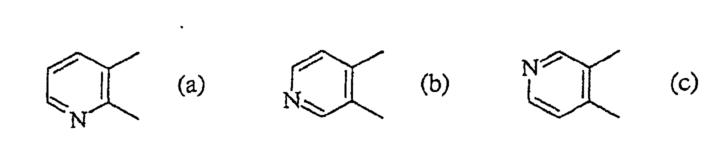

Besides, the substituent of the pyridine ring is selected from, a lower alkyl group, a halogen atom, a cyano group, a trifluoromethyl group, a nitro group, an amino group, a mono-lower alkylamino group, a di-lower alkylamino group, a hydroxy group, a lower alkoxy group, a lower alkylthio group, a lower alkylsulfinyl group, and a lower alkylsulfonyl group.

The term "lower" in the present invention means that alkyl moiety described with "lower" is a lower alkyl group, and the lower alkyl group means an alkyl group having 1 to 6 carbon atoms such as methyl, ethyl, propyl, 2-propyl, butyl, pentyl, hexyl, etc. The halogen atom is fluorine atom, chlorine atom, bromine atom, and iodine atom. The substituted pyridine ring has one or more substituents which are the same or different.

The alkyl group or the alkyl moiety of the substituted alkyl group for R² includes, for example, a straight chain or branched chain alkyl group having 1 to 8 carbon atoms, such as methyl, ethyl, propyl, 2-propyl, butyl, 2-butyl, 2-methylpropyl, 1,1-dimethylethyl, 3-pentyl, 3-hexyl, 4-heptyl, 4-octyl, etc.

The alkenyl group or the alkenyl moiety of the substituted alkenyl group for R² includes, for example, a straight chain or branched chain alkenyl group having 2 to 8 carbon atoms, such as vinyl, allyl, 2-propenyl, 2-butenyl, 3-methyl-2-butenyl, 3-hexenyl, 3-ethyl-2-pentenyl, 4-ethyl-3-hexenyl, etc.

The aromatic group includes, for example, an aryl group and a heteroaryl group.

The aryl group includes, for example, an aryl group having carbon atoms of less than 10, such as phenyl group, naphthyl group, etc.

The heteroaryl group includes, for example, a 5- to 6-membered heteromonocyclic group having 1 to 2 nitrogen atoms, a 5- to 6-membered heteromonocyclic group having 1 to 2 nitrogen atoms and one oxygen atom or one sulfur atom, a 5-membered heteromonocyclic group having one oxygen atom or one sulfur atom, a heterobicyclic group formed by fusing a 6-membered ring and a 5- or 6-membered ring and having 1 to 4 nitrogen atoms, such as 2-pyridyl, 3-pyridyl, 4-pyridyl, 2-thienyl, 3-thienyl, 3-oxadiazolyl, 1-imidazolyl, 2-imidazolyl, 2-thiazolyl, 3-isothiazolyl, 2-oxazolyl, 3-isoxazolyl, 2-furyl, 3-furyl, 3-pyrrolyl, 8-quinolyl, 2-quinazolinyl, 8-purinyl, etc.

The substituted aromatic group has one or more substituents which are the same or different, and the substituents are, for example, a halogen atom, a cyano group, a trifluoromethyl group, a nitro group, a hydroxy group, a methylenedioxy group, a lower alkyl group, a lower alkoxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, a lower alkanoylamino group, a lower alkylsulfonamido group or a group of the formula: -D¹-E-F (D¹ is a direct bond, an oxygen atom, a sulfur atom or a group of the formula: -NR³- (R³ is a hydrogen atom or a lower alkyl group), E is a divalent hydrocarbon group having 1 to 6 carbon atoms and optionally containing an unsaturated bond, or a phenylene group, F is a hydroxy group, a carboxyl group, a lower alkoxycarbonyl group, a benzyloxycarbonyl group, a halogen atom, a cyano group, a benzyloxy group, a lower alkoxy group, a lower alkanoyloxy group, a lower alkylthio group, a lower alkylsulfinyl group, a lower alkylsulfonyl group, a lower alkanoylamino group, a lower alkylsulfonamido group, a phthalimido group, a heteroaryl group, a group of the formula: -NR⁴R⁵ (R⁴ and R⁵ are independently a hydrogen atom or a lower alkyl group, or R⁴ and R⁵ may combine with the adjacent nitrogen atom to which they bond to form a saturated 5- to 7-membered cyclic amino group optionally having one -NR⁸- (R⁸ is a hydrogen atom, a lower alkyl group, a phenyl group, or a benzyl group), or one oxygen atom in the cycle thereof), or a group of the formula: -C(=O)NR⁴R⁵ (R⁴ and R⁵ are the same as defined above)).

The divalent hydrocarbon group having 1 to 6 carbon atoms and optionally containing an unsaturated bond includes, for example, an alkylene group such as methylene, ethylene, trimethylene, tetramethylene, pentamethylene, hexamethylene, etc., an alkenylene group such as propenylene, etc., an alkynylene group such as propynylene, etc.

The heteroaryl group for F includes, for example, a 5- to 6-membered cyclic group having 1 to 3 nitrogen atoms, or a 5-membered cyclic group having one oxygen atom or one sulfur atom, such as 2-pyridyl, 3-pyridyl, 4-pyridyl, 1-pyrrolyl, 1-imidazolyl, 1-(1,2,4-triazolyl), 2-thienyl, 3-thienyl, 2-furyl, 3-furyl, etc. These heteroaryl groups may optionally be substituted by one or more lower alkyl groups which are the same or different. The cyclic amino group formed by NR⁴R⁵ includes, for example, a group represented by the formula: 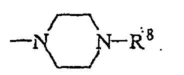 (wherein R⁸ is the same as defined above) such as 4-lower alkyl-1-piperazinyl, 4-phenyl-1-piperazinyl, 4-benzyl-1-piperazinyl, etc., or 1-pyrrolidinyl, 1-piperidinyl, 1-homopiperidinyl, 4-morpholinyl, etc.

The substituted alkyl group, and, the substituted alkenyl group, have one or more substituents which are the same or different, and the substituents are, for example, a halogen atom, a cyano group, a benzyloxy group, a trifluoromethyl group, a hydroxy group, a lower alkoxy group, a lower alkanoyloxy group, an amino group, a mono-lower alkylamino group, a di-lower alkylamino group, a carbamoyl group, a lower alkylaminocarbonyl group, a di-lower alkylaminocarboxyl group, a lower alkoxycarbonylamino group, a carboxyl group, a lower alkoxycarbonyl group, a lower alkylthio group, a lower alkylsulfinyl group, a lower alkylsulfonyl group, a lower alkanoylamino group, a lower alkylsulfonamido group, a phthalimido group, a heteroaryl group, a saturated heterocyclic group or a group of the formula: -D²-E-F (D² is an oxygen atom, a sulfur atom or a group of the formula: -NR³ (R³ is the same as defined above), E and F are the same as defined above). The heteroaryl group is the same heteroaryl groups for the above-mentioned F. The saturated heterocyclic group includes, for example, a 5- to 8-membered cyclic group having one nitrogen atom, a 6- to 8-membered cyclic group having two nitrogen atoms, and a 6- to 8-membered cyclic group having one nitrogen atom and one oxygen atom, such as 1-piperidinyl, 1-pyrrolidinyl, etc.

The substituted alkyl group includes an alkyl group having 1 to 6 carbon atoms which is substituted by a cycloalkyl group or a substituted cycloalkyl group, or an aralkyl group or a substituted aralkyl group.

The aralkyl group or the substituted aralkyl group includes an alkyl group having 1 to 6 carbon atoms which is substituted by the above-mentioned aryl group or substituted aryl group, for example, benzyl, 1-phenylethyl, 2-phenylethyl, 2-naphthylmethyl, etc.

Y is, a phenyl group may optionally be substituted. The substituted phenyl has one or more substituents which are the same or different, and the preferable substituents are, for example, a halogen atom such as fluorine atom, chlorine atom, etc., a cyano group, a trifluoromethyl group, a nitro group, a hydroxy group, methylenedioxy group, a lower alkyl group, a lower alkoxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, a lower alkanoylamino group, a lower alkylsulfonamido group or a group of the formula: -D¹-E-F (D¹, E and F are the same as defined above). The preferable groups for E are, for example, an alkylene chain having 1 to 6 carbon atoms. The preferable groups for F are, for example, a hydroxy group, a halogen atom, a cyano group, a lower alkoxy group, a lower alkanoyloxy group, a lower alkylthio group, a lower alkylsulfinyl group, a lower alkylsulfonyl group, a lower alkanoylamino group, a lower alkylsulfonamido group, a heteroaryl group, or a group of the formula: -NR⁴R⁵ (R⁴ and R⁵ are the same as defined above). The heteroaryl group is, for example, 2-pyridyl, 3-pyridyl, 4-pyridyl, 1-imidazolyl, 1-(1,2,4-triazolyl), etc. The group of the formula: -NR⁴R⁵ (R⁴ and R⁵ are the same as defined above) includes, for example, dimethylamino, diethylamino, piperidinyl, etc.

The preferable groups for B are a phenyl group or heteroaryl group which may optionally be substituted, and the more preferable groups for B are a phenyl group or pyridyl group which is substituted by 1 to 3 groups selected from a halogen atom such as a fluorine atom, chlorine atom, etc., a lower alkyl group, a lower alkoxy group and a lower alkylthio group, for example, 2,6-diisopropylphenyl, 2,4,6-trimethylphenyl, 2,4,6-trimethoxyphenyl, 2,4-difluorophenyl, 2,4,6-trifluorophenyl, 2,6-dimethylthio-3-pyridyl, 2,6-dimethylthio-4-methyl-3-pyridyl, etc.

X is a groups of the following formula:

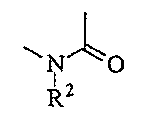

The preferable groups for R² are, an alkyl group, or a substituted alkyl group. The substituted alkyl group has one or more substituents which are the same or different, and the preferable substituents are, for example, a halogen atom such as fluorine atom, chlorine atom, etc., a cyano group, a benzyloxy group, a hydroxy group, a lower alkoxy group, a lower alkanoyloxy group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, an aryl group, a lower alkanoylamino group, a lower alkylsulfonamido group, a phthalimido group, a heteroaryl group, a saturated heterocyclic group, etc. The more preferable substituents are, for example, a fluorine atom, a chlorine atom, a cyano group, a hydroxy group, a carbamoyl group, a 2-pyridyl group, a 3-pyridyl group, a 4-pyridyl group, etc.

The acid for forming an acid addition salt includes, for example, inorganic acids such as hydrochloric acid, hydrobromic acid, hydroiodic acid, sulfuric acid, etc., or organic acids such as acetic acid, oxalic acid, citric acid, malic acid, tartaric acid, fumaric acid, maleic acid, methanesulfonic acid, etc.

The compounds of the present invention may have a stereoisomer due to an asymmetric carbon atom thereof. In such cases, the present compounds also include each isomer or a mixture thereof.

The present compounds and an acid addition salt may be in the form of an anhydrous crystal thereof, or in the form of a solvate thereof such as hydrate.

The compounds of the above-mentioned formula (1) or an acid addition salt thereof can be administered either parenterally or orally when used as the above-mentioned drug. The present compounds can be prepared into liquid preparations such as solutions, emulsions, suspensions, etc., and can be administered in the form of an injection, and if necessary, buffering agents, solubilizers and isotonic agents may be added thereto. The present compounds can also be administered rectally in the form of a suppository. The present compounds can also be administered orally in the form of a conventional administration form such as tablets, capsules, syrups, and suspension. These pharmaceutical preparations can be formulated by mixing an active ingredient with conventional carriers or diluents, binding agents or stabilizers by a conventional manner.

The dosage, the frequency of administration of the present compounds may vary according to the conditions, ages, weights of the patients and the administration form, etc., but the present compounds can be administered in a dosage of 1 to about 500 mg per an adult per day, once a day, or divided into 2 - 4 dosage units.

The naphthyridine derivative which is an active ingredient of the present invention may be prepared by the following processes.

The compound (1) wherein Z is -NH- may be prepared by the following process. 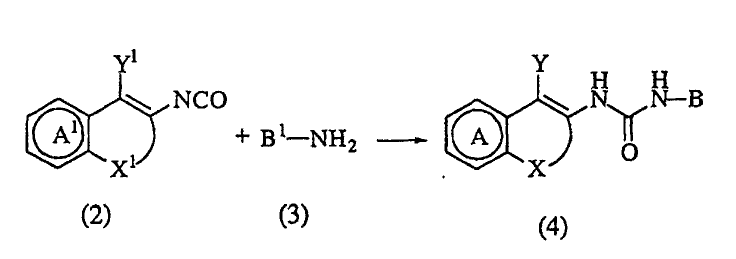 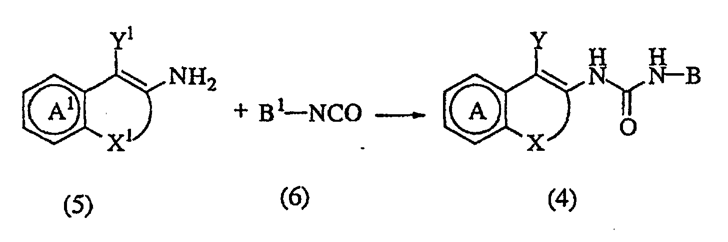 wherein Ring A, X, Y and B are the same as defined above, Ring A¹ is the same groups for Ring A but when these groups contain a reactive group as a substituent such as an amino group, an alkylamino group, a hydroxy group, etc. then these reactive groups should be protected, X¹ is the same groups for X, but when these groups contain a reactive group as a substituent such as an amino group, an alkylamino group, a hydroxy group, a carboxyl group, etc., then these reactive groups should be protected, Y¹ is the same groups for Y, but when these groups contain a reactive group as a substituent such as an amino group, an alkylamino group, a hydroxy group, a carboxyl group, etc., then these reactive groups should be protected, and B¹ is the same groups for B but when these groups contain a reactive group as a substituent such as an amino group, an alkylamino group, a hydroxy group, carboxyl group, etc., then these reactive groups should be protected.

The isocyanate derivative (2) and the amine derivative (3) or an acid addition salt thereof are usually reacted in a solvent at a temperature of from 0°C to a boiling point of the solvent, preferably at a temperature of from room temperature to 120°C, and if necessary, the protecting groups of the product are removed to give the urea derivative (4). The solvent may be any solvent which does not disturb the reaction, but preferably ethers (e.g., ethyl ether, isopropyl ether, tetrahydrofuran, dioxane, etc), aromatic hydrocarbons (e.g., benzene, toluene, etc.), esters (e.g., methyl acetate, ethyl acetate, etc.), N,N-dimethylformamide, dimethylsulfoxide, and the like.

When the amine derivative (3) is used in the form of an acid addition salt thereof, the reaction may preferably proceed by converting the compound (3) into a free form, if necessary. In this case, an agent for converting the compound (2) into a free form is preferably a tertiary amine such as triethylamine, etc., or an aromatic amine such as pyridine, etc. On the other hand, the amine derivative (5) or an acid addition salt thereof and the isocyanate derivative (6) are reacted to give the urea derivative (4), as well.

The protecting groups for amino group, alkylamino group, hydroxy group, carboxyl group, etc., may be conventional protecting groups which are used in the field of the organic chemistry, for example, the protecting group for hydroxy group may be benzyl group, acetyl group, etc., and the protecting group for amino group may be benzyl group, etc., and these protecting groups may be introduced and removed by a conventional method, such as by a method disclosed in Protective Groups in Organic Synthesis, 2nd ed., John Wiley & Sons, Inc.; New York. 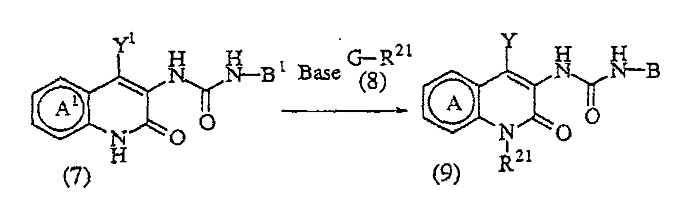 wherein Ring A, Y, B, Ring A¹, Y¹, B¹ are the same as defined above, R²¹ is the same groups for R² but when these groups contain a reactive group as a substituent such as an amino group, an alkylamino group, a hydroxy group, carboxyl group, etc., then these reactive groups should be protected, and G is a leaving group.

Among the urea derivatives (4), the compound of the formula (7) is reacted with an alkylating agent of the formula (8), and if necessary, the protecting groups of the product are removed to give the urea derivative of the formula (9). The alkylation reaction is carried out in a solvent at a temperature of from 0°C to 100°C, preferably at a temperature of from room temperature to 70°C in the presence of a base. The solvent includes, for example, ethers (e.g., tetrahydrofuran, dioxane), ketones (e.g., acetone, 2-butanone), aromatic hydrocarbons (e.g., benzene, toluene), N,N-dimethylformamide, etc. The base includes, for example, sodium hydride, potassium carbonate, sodium carbonate, triethylamine, etc. The leaving group represented by G is usually a halogen atom such as chlorine atom, bromine atom, iodine atom, etc.

The substituents of Ring A, Y, X or B in the urea derivative (4) thus obtained can be converted into others, if necessary. For example, a lower alkylthio group can be converted into a lower alkylsulfonyl group by oxidization. A nitro group is converted into an amino group by reduction. An amino group can be alkylated to a mono- or di-alkyl compound, or an amino group can also be acylated. A 3-chloropropoxy group is converted into a 3-(1-imidazolyl)propoxy group. Such conversion reactions can be carried out by using a well-known technique which is usually applied in the organic chemistry field. As one of the conversion reactions of the substituents, the alkylation reaction can be carried out as follows. 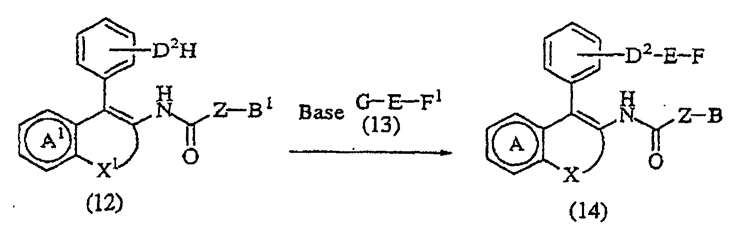 wherein Ring A, B, X, Z, E, F, G, D², Ring A¹, B¹, X¹ are the same as defined above, F¹ is the same groups for F but when these groups contain a reactive group as a substituent such as an amino group, an alkylamino group, a hydroxy group, carboxyl group, etc., then these reactive groups should be protected.

The compound (12) is reacted with the alkylating agent (13) in a solvent, and if necessary, the protecting groups of the product are removed, to give the compound (14). The reaction is usually carried out at a temperature of from 0°C to 100°C, preferably at a temperature of from room temperature to 70°C, in the presence of a base. The solvent may be, for example, ethers (e.g., tetrahydrofuran, dioxane, etc.), aromatic hydrocarbons (e.g., benzene, toluene, etc.), ketones (e.g., acetone, 2-butanone, etc.), dimethylformamide, etc. The base may be, for example, sodium hydride, potassium carbonate, sodium carbonate, triethylamine, etc. When potassium carbonate or sodium carbonate is used, the efficiency of the reaction is optionally increased by addition of sodium iodide or potassium iodide. The leaving group represented by G is usually halogen atoms such as chlorine atom, bromine atom, iodine atom, etc.

The starting compound (2) or (5) for preparing the compound (1) of the present invention or an acid addition salt thereof can be prepared by the following process or by a modified process thereof. 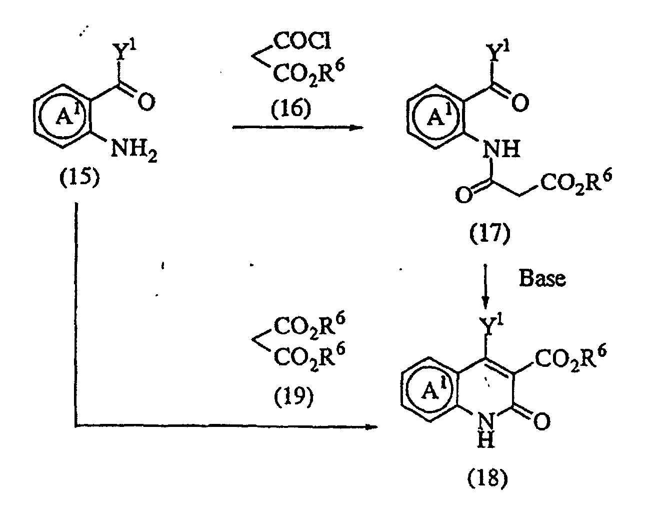 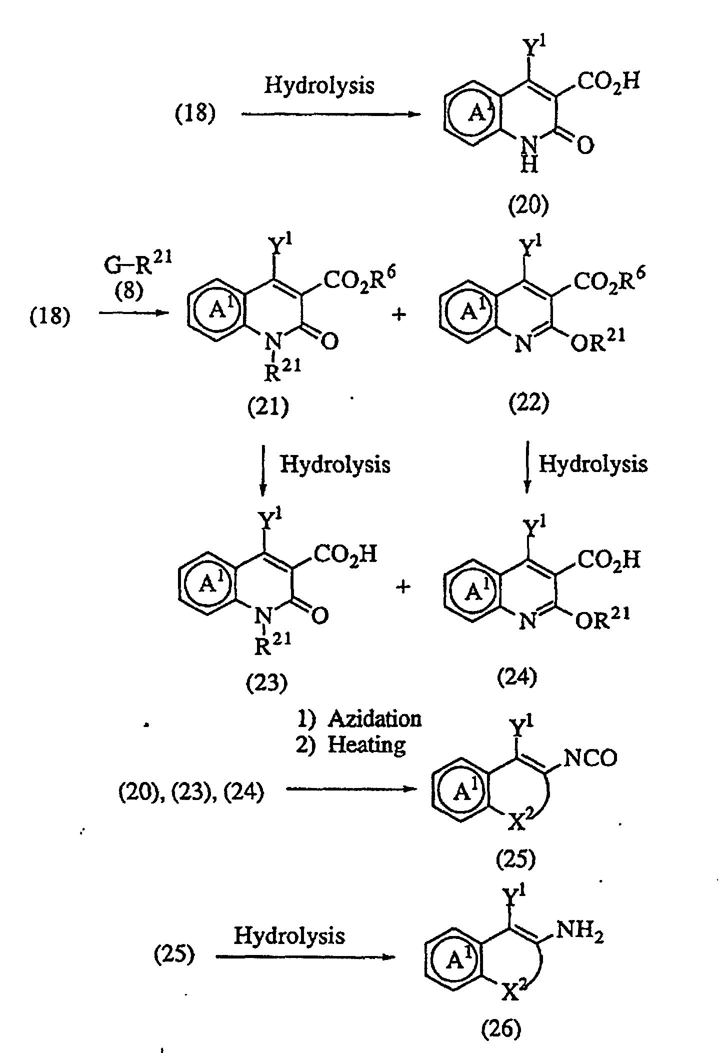 wherein Ring A¹, Y¹, R²¹, and G are the same as defined above, R⁶ is a lower alkyl group, and X² is -NH-CO-, -NR²¹-CO- or -N=C(OR²¹)-.

The starting compound (15) is prepared by the method disclosed in the literature, for example, J. Heterocyclic Chem., **26**, 105-112, 1989, or a modified method thereof. The lower alkyl group represented by R⁶ is preferably one having 1 to 4 carbon atoms, such as methyl, ethyl, propyl, isopropyl, butyl, etc.

The aminoketone derivative (15) is reacted with the acid chloride (16) in the presence of a base in a solvent at a temperature of from -20°C to 150°C, preferably at a temperature of from 0°C to 120°C, to give the amide derivative (17). The solvent may be, for example, ethers (e.g., ethyl ether, tetrahydrofuran, dioxane, etc.), aromatic hydrocarbons (e.g., benzene, toluene, etc.), halogenated solvents (e.g., dichloromethane, chloroform, etc.), pyridine, N,N-dimethylformamide, etc. The base includes, for example, triethylamine, pyridine, potassium carbonate, sodium carbonate, sodium hydrogen carbonate, etc. The amide derivative (17) thus obtained is subjected to cyclization reaction in a solvent such as benzene, toluene, tetrahydrofuran, dimethoxyethane, etc., at a temperature of from 0°C to 200°C, preferably at a temperature of from room temperature to 170°C, in the presence of a base in an amount of 0.1 to 3 mole equivalents, preferably in an amount of 0.1 to 2 mole equivalents to give the compound (18). The base includes, for example, potassium t-butoxide, sodium methoxide, sodium ethoxide, piperidine, triethylamine, 1,5-diazabicyclo[4.3.0]-non-5-ene (DBN), 1,8-diazabicyclo[5.4.0]-7-undecene (DBU), and 1,4-diazabicyclo[2.2.2]octane (DABCO). The compound (18) can also be prepared by heating the compound (15) with the malonic acid diester derivative (19) in the presence of an amine (e.g., piperidine, pyrrolidine, triethylamine, pyridine, DBN, DBU, DABCO, etc.), or potassium fluoride, tetrabutylammonium fluoride, at a temperature of from 60°C to 200°C, without a solvent.

On the other hand, the compound (18) is reacted with the alkylating agent (8) in the presence of a base at a temperature of from 0°C to 150°C, preferably at a temperature of from room temperature to 100°C, in a solvent, to give the N-alkyl compound (21) and/or the O-alkyl compound (22). The solvent includes, for example, alcohols (e.g., methanol, ethanol, etc.), ethers (e.g., tetrahydrofuran, dioxane, etc.), ketones (e.g., acetone, 2-butanone, etc.), and dimethylformamide. The base includes, for example, sodium hydroxide, potassium hydroxide, sodium methoxide, sodium ethoxide, potassium t-butoxide, sodium hydride, potassium carbonate, sodium carbonate, triethylamine, etc. The leaving group represented by G is usually a halogen atom such as chlorine atom, bromine atom, iodine atom, etc. In this reaction, there is obtained a mixture of the compound (21) and the compound (22), but both compounds can be separated by recrystallization or chromatography. On the other hand, the compound (21) can be preferentially obtained by selecting the kinds of the compound (18), the kinds of the solvents, the kinds of the base, or reaction temperature.

The hydrolysis of the compound (18), the compound (21) and the compound (22) is carried out by a conventional method, for example, in a solvent such as methanol, ethanol, isopropanol, tetrahydrofuran, dioxane, dimethoxyethane, etc., at a temperature of from 0°C to 150°C, preferably at a temperature of from 0°C to 100°C, by using a hydroxide of an alkali metal or an alkaline earth metal such as sodium hydroxide, potassium hydroxide, barium hydroxide, etc. The carboxylic acid derivative of the formulae (20), (23) or (24) can be converted into the isocyanate derivative (25) by a conventional method, and if necessary, the compound (25) is further converted into the amine derivative (26). For example, the carboxylic acid derivative of the formulae (20), (23) or (24) is converted into an acid azide compound by using an azidation agent (e.g., diphenylphosphoryl azide (DPPA), etc.) in an amount of 1 to 3 mole equivalents, in the presence of a base (e.g., triethylamine, N-methylmorpholine, etc.), at a temperature of from 0°C to 150°C, preferably at a temperature of from room temperature to 120°C in a solvent, and the acid azide compound thus obtained is heated at a temperature of from 20°C to 200°C, preferably at a temperature of from 30°C to 150°C without isolating from the reaction system, to give the compound (25). Moreover, the compound (25) is hydrolyzed in the same manner as in the hydrolysis of the compound (18), (21) or (22), to give the compound (26).

Some of the compounds (5) can be prepared by the following process, or by a modified process thereof. 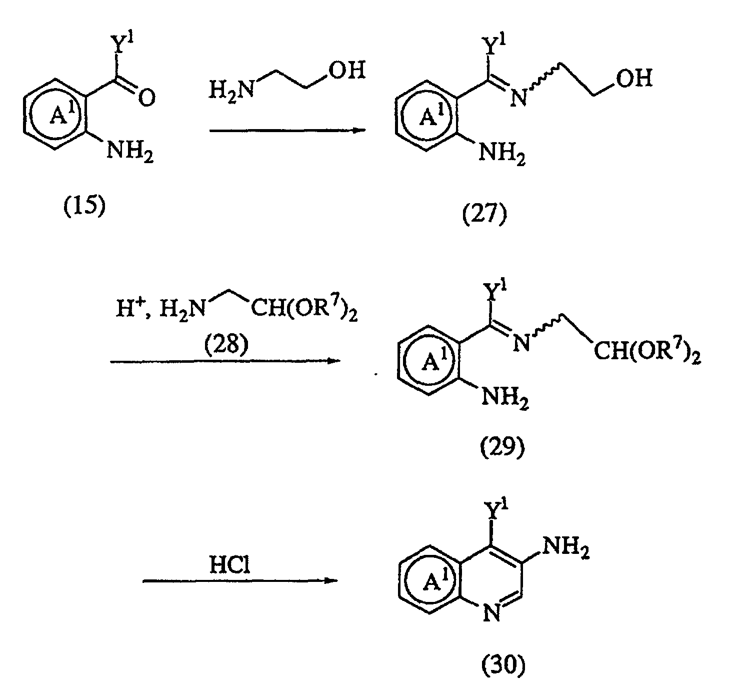 wherein Ring A¹ and Y¹ are the same as defined above, and R⁷ is an alkyl group.

The alkyl group for R⁷ is preferably ones having 1 to 4 carbon atoms such as methyl, ethyl, propyl, isopropyl, butyl, etc.

The aminoketone derivative (15) is treated in the same manner as disclosed in Yakugaku Zasshi, vol. 93, p. 1263 (1973), or a modified method thereof, to give the aminonaphthyridine derivative (30).

The present compounds obtained by the present process, and the intermediates thereof may be purified by a conventional method, for example, column chromatography, recrystallization, etc. The solvent for recrystallization may be, for example, alcohols (e.g., methanol, ethanol, 2-propanol, etc.), ethers (e.g., diethyl ether, etc.), esters (e.g., ethyl acetate, etc.), aromatic solvents (e.g., toluene, etc.), ketones (e.g., acetone, etc.), hydrocarbons (e.g., hexane, etc.), or a mixture of these solvents, which is selected according to the kinds of the compound to be recrystallized.

The representatives of the present compound obtained by the above process are as follows:
N-[4-(3-methoxyphenyl)-1,2-dihydro-2-oxo-1,8-naphthyridin-3-yl]-N'-(2,6-diisopropylphenyl)urea
N-(1-methyl-4-phenyl-1,2-dihydro-2-oxo-1,8-naphthyridin-3-yl)-N'-(2,6-diisopropylphenyl)urea
N-[1-methyl-4-(2-chlorophenyl)-1,2-dihydro-2-oxo-1,8-naphthyridin-3-yl]-N'-(2,6-diisopropylphenyl)urea
N-[1-methyl-4-(2-methoxyphenyl)-1,2-dihydro-2-oxo-1,8-naphthyridin-3-yl]-N'-(2,6-diisopropylphenyl)urea
N-[1-methyl-4-(3-methoxyphenyl)-1,2-dihydro-2-oxo-1,8-naphthyridin-3-yl]-N'-(2,6-diisopropylphenyl)urea
N-[1-methyl-4-(4-methoxyphenyl)-1,2-dihydro-2-oxo-1,8-naphthyridin-3-yl]-N'-(2,6-diisopropylphenyl)urea
N-[1-methyl-4-(2-hydroxyphenyl)-1,2-dihydro-2-oxo-1,8-naphthyridin-3-yl]-N'-(2,6-diisopropylphenyl)urea
N-[1-methyl-4-(3-hydroxyphenyl)-1,2-dihydro-2-oxo-1,8-naphthyridin-3-yl]-N'-(2,6-diisopropylphenyl)urea
N-[1-methyl-4-(4-hydroxyphenyl)-1,2-dihydro-2-oxo-1,8-naphthyridin-3-yl]-N'-(2,6-diisopropylphenyl)urea
N-[1-methyl-4-(2-aminophenyl)-1,2-dihydro-2-oxo-1,8-naphthyridin-3-yl]-N'-(2,6-diisopropylphenyl)urea
N-[1-methyl-4-(3-aminophenyl)-1,2-dihydro-2-oxo-1,8-naphthyridin-3-yl]-N'-(2,6-diisopropylphenyl)urea
N-[1-methyl-4-(4-aminophenyl)-1,2-dihydro-2-oxo-1,8-naphthyridin-3-yl]-N'-(2,6-diisopropylphenyl)urea
N-[1-methyl-4-{3-(2-hydroxyethoxy)phenyl}-1,2-dihydro-2-oxo-1,8-naphthyridin-3-yl]-N'-(2,6-diisopropylphenyl)urea
N-[1-methyl-4-{3-(2-acetoxyethoxy)phenyl}-1,2-dihydro-2-oxo-1,8-naphthyridin-3-yl]-N'-(2,6-diisopropylphenyl)urea
N-[1-methyl-4-{3-(2-diethylaminoethoxy)phenyl}-1,2-dihydro-2-oxo-1,8-naphthyridin-3-yl}-N'-(2,6-diisopropylphenyl)urea
N-[1-methyl-4-{3-(3-hydroxypropoxy)phenyl}-1,2-dihydro-2-oxo-1,8-naphthyridin-3-yl]-N'-(2,6-diisopropylphenyl)urea
N-[1-methyl-4-{3-(3-acetoxypropoxy)phenyl}-1,2-dihydro-2-oxo-1,8-naphthyridin-3-yl]-N'-(2,6-diisopropylphenyl)urea
N-[ 1-methyl-4-{3-(3-dimethylaminopropoxy)phenyl}-1,2-dihydro-2-oxo-1,8-naphthyridin-3-yl]-N'-(2,6-diisopropylphenyl)urea
N-[1-methyl-4-[3-{3-(4-phenyl-1-piperazinyl)propoxy}phenyl]-1,2-dihydro-2-oxo-1,8-naphthyridin-3-yl]-N'-(2,6-diisopropylphenyl)urea
N-[1-methyl-4-{3-(2-piperidinoethoxy)phenyl}-1,2-dihydro-2-oxo-1,8-naphthyridin-3-yl]-N'-(2,6-diisopropylphenyl)urea
N-[1-carboxymethyl-1,2-dihydro-4-(3-methoxyphenyl)-2-oxo-1,8-naphthyridin-3-yl]-N'-(2,6-diisopropylphenyl)urea
N-[1-tert-butoxycarbonylmethyl-4-(3-methoxyphenyl)-1,2-dihydro-2-oxo-1,8-naphthyridin-3-yl]-N'-(2,6-diisopropylphenyl)urea
N-[1-(4-pyridylmethyl)-4-(3-methoxyphenyl)-1,2-dihydro-2-oxo-1,8-naphthyridin-3-yl]-N'-(2,6-diisopropylphenyl)urea
N-[1-(2-pyridylmethyl)-4-(3-methoxyphenyl)-1,2-dihydro-2-oxo-1,8-naphthyridin-3-yl]-N'-(2,6-diisopropylphenyl)urea
N-[1-ethyl-4-(3-methoxyphenyl)-1,2-dihydro-2-oxo-1,8-naphthyridin-3-yl]-N'-(2,6-diisopropylphenyl)urea
N-[1-(2-methoxyethyl)-4-(3-methoxyphenyl)-1,2-dihydro-2-oxo-1,8-naphthyridin-3-yl]-N'-(2,6-diisopropylphenyl)urea
N-[1-(2-hydroxyethyl)-4-(3-methoxyphenyl)-1,2-dihydro-2-oxo-1,8-naphthyridin-3-yl]-N'-(2,6-diisopropylphenyl)urea
N-[1-(2-cyanoethyl)-4-(3-methoxyphenyl)-1,2-dihydro-2-oxo-1,8-naphthyridin-3-yl]-N'-(2,6-diisopropylphenyl)urea
N-[1-(2-diethylaminoethyl)-4-(3-methoxyphenyl)-1,2-dihydro-2-oxo-1,8-naphthyridin-3-yl]-N'-(2,6-diisopropylphenyl)urea
N-[1-propyl-4-(3-methoxyphenyl)-1,2-dihydro-2-oxo-1,8-naphthyridin-3-yl)-N'-(2,6-diisopropylphenyl)urea
N-[1-(3-cyanopropyl)-4-(3-methoxyphenyl)-1,2-dihydro-2-oxo-1,8-naphthyridin-3-yl]-N'-(2,6-diisopropylphenyl)urea
N-[1-(3-cyanopropyl)-4-(3-hydroxyphenyl)-1,2-dihydro-2-oxo-1,8-naphthyridin-3-yl]-N'-(2,6-diisopropylphenyl)urea
N-[1-(3-cyanopropyl)-4-{3-(2-hydroxyethoxy)phenyl}-1,2-dihydro-2-oxo-1,8-naphthyridin-3-yl]-N'-(2,6-diisopropylphenyl)urea
N-[1-(3-cyanopropyl)-4-{3-(2-diethylaminoethoxy)phenyl}-1,2-dihydro-2-oxo-1,8-naphthyridin-3-yl]-N'-(2,6-diisopropylphenyl)urea
N-[1-(3-cyanopropyl)-4-{3-(3-hydroxypropoxy)phenyl}-1,2-dihydro-2-oxo-1,8-naphthyridin-3-yl]-N'-(2,6-diisopropylphenyl)urea
N-[1-(3-cyanopropyl)-4-[3-{3-(1-imidazolyl)propoxy}phenyl]-1,2-dihydro-2-oxo-1,8-naphthyridin-3-yl]-N'-(2,6-diisopropylphenyl)urea
N-[1-(3-hydroxypropyl)-4-(3-methoxyphenyl)-1,2-dihydro-2-oxo-1,8-naphthyridin-3-yl]-N'-(2,6-diisopropylphenyl)urea
N-[1-(3-benzyloxypropyl)-4-(3-methoxyphenyl)-1,2-dihydro-2-oxo-1,8-naphthyridin-3-yl]-N'-(2,6-diisopropylphenyl)urea
N-[1-(3-aminopropyl)-4-(3-methoxyphenyl)-1,2-dihydro-2-oxo-1,8-naphthyridin-3-yl]-N'-(2,6-diisopropylphenyl)urea
N-[1-(3-dimethylaminopropyl)-4-(3-methoxyphenyl)-1,2-dihydro-2-oxo-1,8-naphthyridin-3-yl]-N'-(2,6-diisopropylphenyl)urea
N-[1-(3-aminocarbonylpropyl)-4-(3-methoxyphenyl)-1,2-dihydro-2-oxo-1,8-naphthyridin-3-yl]-N'-(2,6-diisopropylphenyl)urea
N-[1-(3-acetylaminopropyl)-4-(3-methoxyphenyl)-1,2-dihydro-2-oxo-1,8-naphthyridin-3-yl]-N'-(2,6-diisopropylphenyl)urea
N-[1-(3-phthalimidopropyl)-4-(3-methoxyphenyl)-1,2-dihydro-2-oxo-1,8-naphthyridin-3-yl]-N'-(2,6-diisopropylphenyl)urea
N-[1-(3-dimethylaminopropyl)-4-(3-methoxyphenyl)-1,2-dihydro-2-oxo-1,8-naphthyridin-3-yl]-N'-(2,6-diisopropylphenyl)urea
N-[1-(3-diethylaminopropyl)-4-(3-methoxyphenyl)-1,2-dihydro-2-oxo-1,8-naphthyridin-3-yl]-N'-(2,6-diisopropylphenyl)urea
N-[1-(3-piperidinopropyl)-4-(3-methoxyphenyl)-1,2-dihydro-2-oxo-1,8-naphthyridin-3-yl]-N'-(2,6-diisopropylphenyl)urea
N-[1-{3-(1-imidazolyl)propyl}-4-(3-methoxyphenyl)-1,2-dihydro-2-oxo-1,8-naphthyridin-3-yl]-N'-(2,6-diisopropylphenyl)urea
N-[1-isopropyl-4-(3-methoxyphenyl)-1,2-dihydro-2-oxo-1,8-naphthyridin-3-yl]-N'-(2,6-diisopropylphenyl)urea
N-[1-butyl-4-(2-methoxyphenyl)-1,2-dihydro-2-oxo-1,8-naphthyridin-3-yl]-N'-(2,6-diisopropylphenyl)urea
N-[1-butyl-4-(3-methoxyphenyl)-1,2-dihydro-2-oxo-1,8-naphthyridin-3-yl]-N'-(2,6-diisopropylphenyl)urea
N-[1-butyl-4-(4-methoxyphenyl)-1,2-dihydro-2-oxo-1,8-naphthyridin-3-yl]-N'-(2,6-diisopropylphenyl)urea
N-[1-butyl-4-(2-hydroxyphenyl)-1,2-dihydro-2-oxo-1,8-naphthyridin-3-yl]-N'-(2,6-diisopropylphenyl)urea
N-[1-butyl-4-(3-hydroxyphenyl)-1,2-dihydro-2-oxo-1,8-naphthyridin-3-yl]-N'-(2,6-diisopropylphenyl)urea
N-[1-butyl-4-(4-hydroxyphenyl)-1,2-dihydro-2-oxo-1,8-naphthyridin-3-yl]-N'-(2,6-diisopropylphenyl)urea
N-[1-butyl-4-(2-aminophenyl)-1,2-dihydro-2-oxo-1,8-naphthyridin-3-yl]-N'-(2,6-diisopropylphenyl)urea
N-[1-butyl-4-(3-aminophenyl)-1,2-dihydro-2-oxo-1,8-naphthyridin-3-yl]-N'-(2,6-diisopropylphenyl)urea
N-[1-butyl-4-(4-aminophenyl)-1,2-dihydro-2-oxo-1,8-naphthyridin-3-yl]-N'-(2,6-diisopropylphenyl)urea
N-[1-butyl-4-{3-(2-pyridylmethoxy)phenyl}-1,2-dihydro-2-oxo-1,8-naphthyridin-3-yl]-N'-(2,6-diisopropylphenyl)urea
N-[1-butyl-4-{3-(3-pyridylmethoxy)phenyl}-1,2-dihydro-2-oxo-1,8-naphthyridin-3-yl]-N'-(2,6-diisopropylphenyl)urea
N-[1-butyl-4-{3-(4-pyridylmethoxy)phenyl}-1,2-dihydro-2-oxo-1,8-naphthyridin-3-yl]-N'-(2,6-diisopropylphenyl)urea
N-[1-butyl-4-{3-(2-hydroxyethoxy)phenyl}-1,2-dihydro-2-oxo-1,8-naphthyridin-3-yl]-N'-(2,6-diisopropylphenyl)urea
N-[1-butyl-4-{3-(2-acetoxyethoxy)phenyl}-1,2-dihydro-2-oxo-1,8-naphthyridin-3-yl]-N'-(2,6-diisopropylphenyl)urea
N-[1-butyl-4-{3-(2-diethylaminoethoxy)phenyl}-1,2-dihydro-2-oxo-1,8-naphthyridin-3-yl]-N'-(2,6-diisopropylphenyl)urea
N-[1-butyl-4-{3-(2-dimethylaminoethoxy)phenyl}-1,2-dihydro-2-oxo-1,8-naphthyridin-3-yl]-N'-(2,6-diisopropylphenyl)urea
N-[1-butyl-4-{3-(2-piperidinoethoxy)phenyl}-1,2-dihydro-2-oxo-1,8-naphthyridin-3-yl]-N'-(2,6-diisopropylphenyl)urea
N-[1-butyl-4-{3-(2-(1-pyrrolidinyl)ethoxy)phenyl}-1,2-dihydro-2-oxo-1,8-naphthyridin-3-yl]-N'-(2,6-diisopropylphenyl)urea
N-[1-butyl-4-{3-(2-morpholinoethoxy)phenyl}-1,2-dihydro-2-oxo-1,8-naphthyridin-3-yl]-N'-(2,6-diisopropylphenyl)urea
N-[1-butyl-4-{3-(3-chloropropoxy)phenyl}-1,2-dihydro-2-oxo-1,8-naphthyridin-3-yl]-N'-(2,6-diisopropylphenyl)urea
N-[1-butyl-4-{3-(3-hydroxypropoxy)phenyl}-1,2-dihydro-2-oxo-1,8-naphthyridin-3-yl]-N'-(2,6-diisopropylphenyl)urea
N-[1-butyl-4-{3-{3-acetoxypropoxy)phenyl}-1,2-dihydro-2-oxo-1,8-naphthyridin-3-yl]-N'-(2,6-diisopropylphenyl)urea
N-[1-butyl-4-{3-(3-benzyloxypropoxy)phenyl}-1,2-dihydro-2-oxo-1,8-naphthyridin-3-yl]-N'-(2,6-diisopropylphenyl)urea
N-[1-butyl-4-{3-(3-phthalimidopropoxy)phenyl}-1,2-dihydro-2-oxo-1,8-naphthyridin-3-yl]-N'-(2,6-diisopropylphenyl)urea
N-[1-butyl-4-{3-(3-aminopropoxy)phenyl}-1,2-dihydro-2-oxo-1,8-naphthyridin-3-yl]-N'-(2,6-diisopropylphenyl)urea
N-[1-butyl-4-{3-(3-dimethylaminopropoxy)phenyl}-1,2-dihydro-2-oxo-1,8-naphthyridin-3-yl]-N'-(2,6-diisopropylphenyl)urea
N-[1-butyl-4-{3-(3-diethylaminopropoxy)phenyl}-1,2-dihydro-2-oxo-1,8-naphthyridin-3-yl]-N'-(2,6-diisopropylphenyl)urea
N-[1-butyl-4-{3-(3-piperidinopropoxy)phenyl}-1,2-dihydro-2-oxo-1,8-naphthyridin-3-yl]-N'-(2,6-diisopropylphenyl)urea
N-[1-butyl-4-[3-{3-(4-phenyl-1-piperazinyl)propoxy}phenyl]-1,2-dihydro-2-oxo-1,8-naphthyridin-3-yl]-N'-(2,6-diisopropylphenyl)urea
N-[1-butyl-4-[3-{3-(1,2,4-triazol-1-yl)propoxy}phenyl]-1,2-dihydro-2-oxo-1,8-naphthyridin-3-yl]-N'-(2,6-diisopropylphenyl)urea
N-[1-butyl-4-[3-{3-(1-imidazolyl)propoxy}phenyl]-1,2-dihydro-2-oxo-1,8-naphthyridin-3-yl]-N'-(2,6-diisopropylphenyl)urea
N-[1-butyl-4-{3-(3-diethylaminopropyl)phenyl}-1,2-dihydro-2-oxo-1,8-naphthyridin-3-yl]-N'-(2,6-diisopropylphenyl)urea
N-[1-butyl-4-{3-(2-diethylaminoethylthio)phenyl}-1,2-dihydro-2-oxo-1,8-naphthyridin-3-yl]-N'-(2,6-diisopropylphenyl)urea
N-[1-pentyl-4-(3-methoxyphenyl)-1,2-dihydro-2-oxo-1,8-naphthyridin-3-yl]-N'-(2,6-diisopropylphenyl)urea
N-[1-(4-methylpentyl)-4-(3-methoxyphenyl)-1,2-dihydro-2-oxo-1,8-naphthyridin-3-yl]-N'-(2,6-diisopropylphenyl)urea
N-[1-methyl-4-(2-chlorophenyl)-1,2-dihydro-2-oxo-1,8-naphthyridin-3-yl]-N'-(2,4-difluorophenyl)urea
N-[1-methyl-4-(2-chlorophenyl)-1,2-dihydro-2-oxo-1,8-naphthyridin-3-yl]-N'-(2,4,6-trimethylphenyl)urea
N-[1-methyl-4-(3-methoxyphenyl)-1,2-dihydro-2-oxo-1,8-naphthyridin-3-yl]-N'-(2,4-difluorophenyl)urea
N-[1-methyl-4-(3-methoxyphenyl)-1,2-dihydro-2-oxo-1,8-naphthyridin-3-yl]-N'-(2,4,6-trimethylphenyl)urea
N-[1-methyl-4-(3-methoxyphenyl)-1,2-dihydro-2-oxo-1,8-naphthyridin-3-yl]-N'-(2-trifluoromethylphenyl)urea
N-[1-methyl-4-(3-methoxyphenyl)-1,2-dihydro-2-oxo-1,8-naphthyridin-3-yl]-N'-(2,6-dichlorophenyl)urea
N-[1-methyl-4-(3-methoxyphenyl)-1,2-dihydro-2-oxo-1,8-naphthyridin-3-yl]-N'-(2-methoxy-4-nitrophenyl)urea
N-[1-methyl-4-(3-methoxyphenyl)-1,2-dihydro-2-oxo-1,8-naphthyridin-3-yl]-N'-(2-isopropylphenyl)urea
N-[1-methyl-4-(3-methoxyphenyl)-1,2-dihydro-2-oxo-1,8-naphthyridin-3-yl]-N'-(2-ethylphenyl)urea
N-[1-methyl-4-(3-methoxyphenyl)-1,2-dihydro-2-oxo-1,8-naphthyridin-3-yl]-N'-(2-isopropyl-6-methylphenyl)urea
N-[1-methyl-4-(3-methoxyphenyl)-1,2-dihydro-2-oxo-1,8-naphthyridin-3-yl]-N'-(2-chloro-3-pyridyl)urea
N-[1-methyl-4-(3-methoxyphenyl)-1,2-dihydro-2-oxo-1,8-naphthyridin-3-yl]-N'-(3,5-dichloro-2-pyridyl)urea
N-[1-methyl-4-(3-methoxyphenyl)-1,2-dihydro-2-oxo-1,8-naphthyridin-3-yl]-N'-(8-quinolyl)urea
N-[1-methyl-4-(2-chlorophenyl)-1,2-dihydro-2-oxo-1,7-naphthyridin-3-yl]-N'-(2,4,6-trimethylphenyl)urea
N-[1-methyl-4-(2-chlorophenyl)-1,2-dihydro-2-oxo-1,7-naphthyridin-3-yl]-N'-(2,6-diisopropylphenyl)urea
N-[1-methyl-4-(3-methoxyphenyl)-1,2-dihydro-2-oxo-1,7-naphthyridin-3-yl]-N'-(2,6-diisopropylphenyl)urea
N-[1-methyl-4-(2-chlorophenyl)-1,2-dihydro-2-oxo-1,6-naphthyridin-3-yl]-N'-(2,6-diisopropylphenyl)urea
N-[1-methyl-4-(2-chlorophenyl)-1,2-dihydro-2-oxo-1,6-naphthyridin-3-yl]-N'-(2,4,6-trimethylphenyl)urea
N-[1-methyl-4-(3-methoxyphenyl)-1,2-dihydro-2-oxo-1,6-naphthyridin-3-yl]-N'-(2,6-diisopropylphenyl)urea
N-[1-isopropyl-4-(3-methoxyphenyl)-1,2-dihydro-2-oxo-1,6-naphthyridin-3-yl]-N'-(2,6-diisopropylphenyl)urea
N-[1-(3-methyl-2-butenyl)-4-{3-(2-diethylaminoethoxy)phenyl}-1,2-dihydro-2-oxo-1,8-naphthyridin-3-yl]-N'-(2,6-diisopropylphenyl)urea
N-[1-(3-pentynyl)-4-{3-(2-diethylaminoethoxy)phenyl}-1,2-dihydro-2-oxo-1,8-naphthyridin-3-yl]-N'-(2,6-diisopropylphenyl)urea
N-[1-benzyl-4-{3-(2-diethylaminoethoxy)phenyl}-1,2-dihydro-2-oxo-1,8-naphthyridin-3-yl]-N'-(2,6-diisopropylphenyl)urea
N-[1-cyclopropylmethyl-4-{3-(2-diethylaminoethoxy)phenyl}-1,2-dihydro-2-oxo-1,8-naphthyridin-3-yl]-N'-(2,6-diisopropylphenyl)urea

### BEST MODE FOR CARRYING OUT THE INVENTION

The present invention is illustrated by Reference Examples and Examples, but should not be construed to be limited thereto.

### Example 1 (not encompassed by the present invention)

### Preparation of N-[4-(2-chlorophenyl)-1,7-naphthyridin-3-yl]-N'-(2,4-difluorophenyl)urea:

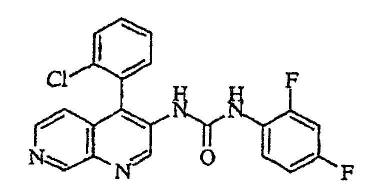

A solution of 3-amino-4-(2-chlorophenyl)-1,7-naphthyridine (196 mg, 0.66 mmol) and 2,4-difluorophenylisocyanate (102 mg, 0.66 mmol) in tetrahydrofuran (50 ml) was refluxed for 8 hours. The mixture was concentrated, and the residue was purified by column chromatography (silica gel; methanol:chloroform = 1:9), and recrystallized from hexane to give the title compound (98 mg, 0.24 mmol) as colorless powder.
m.p. 213-216°C
¹H-NMR δ (DMSO-d₆) 10.52 (1H, br), 8.25 (1H, d, J=5.0Hz), 8.04-8.08 (2H, m), 7.59-7.75 (2H, m), 7.17-7.46 (4H, m), 6.91-7.00 (1H, m), 6.67-6.74 (0.5H, m), 6.62 (1H, d, J=5.0Hz), 6.30-6.36 (0.5H, m)
IR (KBr) 1683, 1596, 1508, 1400 cm⁻¹

### Example 2

### Preparation of N-[1-methyl-4-(2-chlorophenyl)-1,2-dihydro-2-oxo-1,8-naphthyridin-3-yl]-N'-(2,4-difluorophenyl)urea

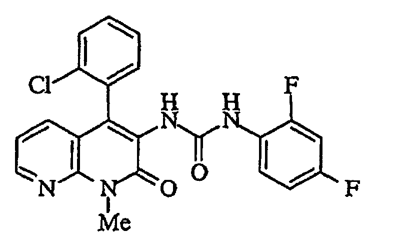

A solution of 1-methyl-4-(2-chlorophenyl)-1,2-dihydro-2-oxo-1,8-naphthyridine-3-carboxylic acid (315 mg, 1 mmol), diphenylphosphoryl azide (330 mg, 1.2 mmol) and triethylamine (101 mg, 1 mmol) in benzene (4 ml) was stirred at room temperature for 0.5 hour, and then refluxed for 0.5 hour. After allowed to stand for cooling, to the mixture was added 2,4-difluoroaniline (152 mg, 1.2 mmol), and the mixture was stirred at room temperature for 0.5 hour, and then stirred under reflux for two hours. After allowed to stand for cooling, the mixture was washed with water, washed with a saturated sodium chloride solution, dried over anhydrous magnesium sulfate, and concentrated under reduced pressure. The resulting solid was recrystallized from isopropanol to give the title compound (288 mg, 0.65 mmol) as a colorless crystal.
m.p. 225-226°C
¹H-NMR δ (CDCl₃) 8.61 (1H, dd, J=4.6Hz, 1.7Hz), 7.66-7.75 (2H, m), 7.38-7.56 (6H, m), 7.16 (1H, dd, J=7.9Hz, 4.6Hz), 6.67-677 (2H, m), 4.00 (3H, s)
IR (KBr) 1715, 1636, 1613, 1584, 1584, 1550, 1500, 1434 cm⁻¹

### Example 3

### Preparation of N-[1-methyl-4-(2-chlorophenyl)-1,2-dihydro-2-oxo-1,8-naphthyridin-3-yl]-N'-(2,4,6-trimethylphenyl)urea

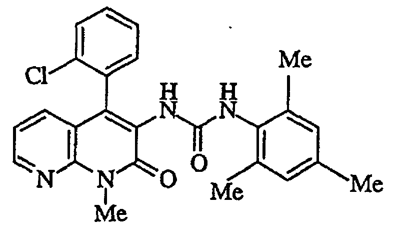

The title compound was obtained in the same manner as in Example 2 from 1-methyl-4-(2-chlorophenyl)-1,2-dihydro-2-oxo-1,8-naphthyridine-3-carboxylic acid and 2,4,6-trimethylaniline.
m.p. >250°C
¹H-NMR δ (CDCl₃) 8.55 (1H, dd, J=4.6Hz, 2.0Hz), 7.37-7.53 (5H, m), 7.10 (1H, dd, J=7.9Hz, 4.6Hz), 6.91 (2H, brs), 6.36 (0.7H, br), 5.73 (0.7H, br), 3.91 (3H, s), 2.27 (6H, brs), 2.02 (3H, brs)
IR (KBr) 3271, 1658, 1634, 1554, 1458, 1118 cm⁻¹

### Example 4

### Preparation of N-[1-methyl-4-(2-chlorophenyl)-1,2-dihydro-2-oxo-1,8-naphthyridin-3-yl]-N'-(2,6-diisopropylphenyl)urea

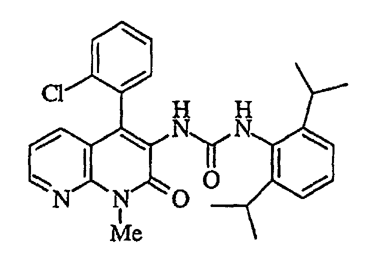

The title compound was obtained in the same manner as in Example 2 from 1-methyl-4-(2-chlorophenyl)-1,2-dihydro-2-oxo-1,8-naphthyridine-3-carboxylic acid and 2,6-diisopropylaniline.
m.p. 193-196°C
¹H-NMR δ (CDCl₃) 8.54 (1H, br), 7.53-7.57 (1H, m), 7.03-7.46 (8H, m), 6.47 (0.7H, brs), 5.65 (0.7H, brs), 3.88 (3H, brs), 3.22 (m) and 2.94 (m) (total 1H), 1.02-1.38 (12H, m)
IR (KBr) 3342, 2963, 1714, 1629, 1608, 1581, 1509, 1461 cm⁻¹

### Example 5

### Preparation of N-[1-methyl-4-(2-chlorophenyl)-1,2-dihydro-2-oxo-1,7-naphthyridin-3-yl]-N'-(2,4,6-trimethylphenyl)urea

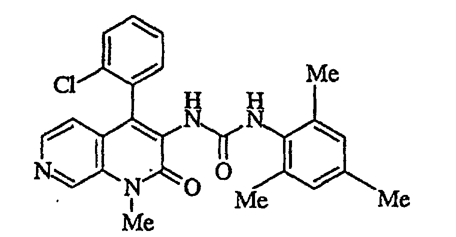

A solution of 1-methyl-4-(2-chlorophenyl)-1,2-dihydro-2-oxo-1,7-naphthyridine-3-carboxylic acid (315 mg, 1 mmol), diphenylphosphoryl azide (330 mg, 1.2 mmol) and triethylamine (101 mg, 1 mmol) in N,N-dimethylformamide (DMF, 5 ml) was stirred at room temperature for 0.5 hour, and stirred at 80-90°C for 0.5 hour. After allowed to stand for cooling, to the mixture was added 2,4,6-trimethylaniline (162 mg, 1.2 mmol), and the mixture was stirred at room temperature for 0.5 hour, and then stirred at 80-90°C for two hours. After allowed to stand for cooling, the mixture was diluted with ethyl acetate, washed with water, washed with a saturated sodium chloride solution, dried over anhydrous magnesium sulfate, and concentrated under reduced pressure. The resulting solid was recrystallized from ethanol to give the title compound (350 mg, 0.78 mmol) as a colorless crystal.
m.p. 222-224°C
¹H-NMR δ (CDCl₃) 8.83 (1H, s), 8.36 (1H, d, J=5.3Hz), 7.50-7.54 (1H, m), 7.38-7.43 (3H, m), 7.02 (1H, d, J=5.3Hz), 6.93 (1H, brs), 6.62 (0.5H, br), 5.68 (0.5H, br), 3.86 (3H, brs), 2.27 (6H, brs), 2.05 (3H, brs)
IR (KBr) 1658, 1638, 1545, 1432 cm⁻¹

### Example 6

### Preparation of N-[1-methyl-4-(2-chlorophenyl)-1,2-dihydro-2-oxo-1,7-naphthyridin-3-yl]-N'-(2,6-diisopropylphenyl)urea

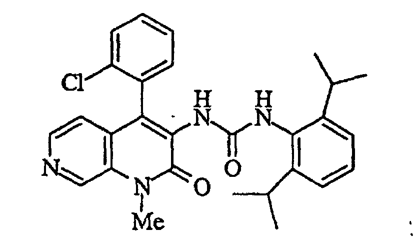

The title compound was obtained in the same manner as in Example 5 from 1-methyl-4-(2-chlorophenyl)-1,2-dihydro-2-oxo-1,7-naphthyridine-3-carboxylic acid and 2,6-diisopropylaniline.
m.p. 153-154°C
¹H-NMR δ (CDCl₃) 8.83 (1H, br), 8.34 (1H, d, J=5.6Hz), 7.01-7.61 (7H, m), 6.97 (1H, d, J=5.6Hz), 6.72 (0.7H, br), 5.80 (0.7H, br), 3.80 (3H, s), 3.21 (1H, m), 2.96 (1H, m), 1.03-1.36 (12H, m)
IR (KBr) 2963, 1645, 1591, 1505 cm⁻¹

### Example 7

### Preparation of N-[1-methyl-4-(3-methoxyphenyl)-1,2-dihydro-2-oxo-1,8-naphthyridin-3-yl]-N'-(2,6-diisopropylphenyl)urea

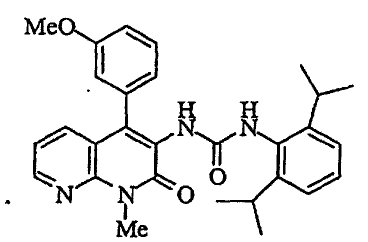

A solution of 1-methyl-4-(3-methoxyphenyl)-1,2-dihydro-2-oxo-1,8-naphthyridine-3-carboxylic acid (315 mg, 1 mmol), diphenylphosphoryl azide (330 mg, 1.2 mmol) and triethylamine (101 mg, 1 mmol) in toluene (4 ml) was stirred at room temperature for 0.5 hour, and then refluxed for 0.5 hour. After allowed to stand for cooling, to the mixture was added 2,6-diisopropylaniline (216 mg, 1.2 mmol), and the mixture was stirred at room temperature for 0.5 hour, and then stirred under reflux for two hours. After allowed to stand for cooling, the mixture was diluted with ethyl acetate, washed with water, washed with a saturated sodium chloride solution, and dried over anhydrous magnesium sulfate. The resultant was concentrated under reduced pressure, and the residue was purified by silica gel column chromatography (ethyl acetate:hexane = 1:1), and crystallized from diethyl ether to give the title compound (302 mg, 0.62 mmol) as a colorless crystal.
m.p. 169-170°C
¹H-NMR δ (CDCl₃) 7.64-7.72 (1H, m), 6.85-7.39 (8H, m), 6.11 (0.5H, br), 5.90 (0.5H, br), 3.92 (s) and 3.86 (s) (total 3H), 3.84 (3H, s), 2.85-3.15 (2H, m), 1.08-1.29 (12H, m)
IR (KBr) 2964, 1716, 1654, 1509 cm⁻¹

### Example 8

### Preparation of N-[4-(3-methoxyphenyl)-1,2-dihydro-2-oxo-1,8-naphthyridin-3-yl]-N'-(2,6-diisopropylphenyl)urea

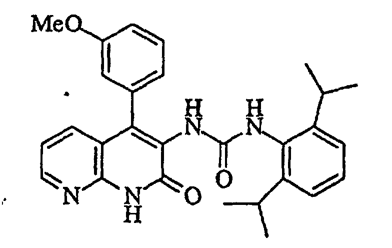

The title compound was obtained in the same manner as in Example 7 from 4-(3-methoxyphenyl)-1,2-dihydro-2-oxo-1,8-naphthyridine-3-carboxylic acid and 2,6-diisopropylaniline.
¹H-NMR δ (CDCl₃) 11.45 (1H, brs), 8.58 (1H, br), 7.65 (1H, br), 6.96-7.40 (7H, m), 6.03 (1H, br), 3.84 (3H, s), 2.96 (br) and 3.18 (br) (total 2H), 1.07-1.20 (12H, br)

### Example 9

### Preparation of N-[1-methyl-4-(4-methoxyphenyl)-1,2-dihydro-2-oxo-1,8-naphthyridin-3-yl]-N'-(2,6-diisopropylphenyl)urea

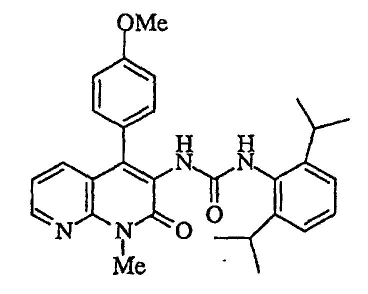

The title compound was obtained in the same manner as in Example 7 from 1-methyl-4-(4-methoxyphenyl)-1,2-dihydro-2-oxo-1,8-naphthyridine-3-carboxylic acid and 2,6-diisopropylaniline.
m.p. 179-182°C

### Example 10

### Preparation of N-[1-isopropyl-4-(3-methoxyphenyl)-1,2-dihydro-2-oxo-1,8-naphthyridin-3-yl]-N'-(2,6-diisopropylphenyl)urea

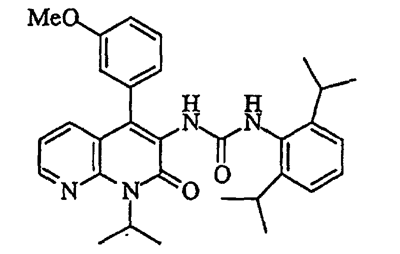

The title compound was obtained in the same manner as in Example 7 from 1-isopropyl-4-(3-methoxyphenyl)-1,2-dihydro-2-oxo-1,8-naphthyridine-3-carboxylic acid and 2,6-diisopropylaniline.
¹H-NMR δ (DMSO-d₆) 8.58 (1H, dd, J=4.6Hz, 2.0Hz), 7.75 (2H, br), 7.62 (1H, dd, J=4.6Hz, 2.0Hz), 7.40 (1H, dd, J=7.9Hz, 7.9Hz), 7.24 (1H, dd, J=7.9Hz, 4.6Hz), 7.13-7.18 (1H, m), 7.00-7.05 (3H, m), 6.91-6.98 (2H, m), 6.10 (1H, br), 3.77 (3H, s), 2.86-2.96 (2H, m), 1.63 (6H, d, J=6.9Hz), 1.08 (12H, br)

### Example 11

### Preparation of N-[1-(2-methoxyethyl)-4-(3-methoxphenyl)-1,2-dihydro-2-oxo-1,8-naphthyridin-3-yl]-N'-(2,6-diisopropylphenyl)urea

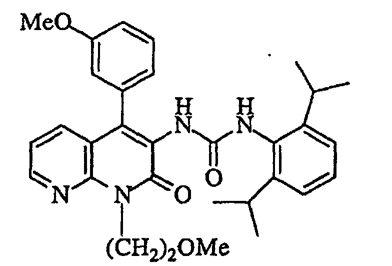

The title compound was obtained in the same manner as in Example 7 from 1-(2-methoxyethyl)-4-(3-methoxyphenyl)-1,2-dihydro-2-oxo-1,8-naphthyridine-3-carboxylic acid and 2,6-diisopropylaniline.
m.p. 164-165°C

### Example 12

### Preparation of N-[1-methyl-4-(3-methoxyphenyl)-1,2-dihydro-2-oxo-1,8-naphthyridin-3-yl]-N'-(2,4-difluorophenyl)urea

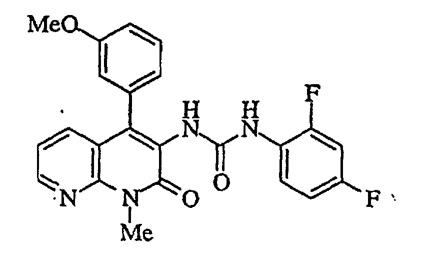

The title compound was obtained in the same manner as in Example 7 from 1-methyl-4-(3-methoxyphenyl)-1,2-dihydro-2-oxo-1,8-naphthyridine-3-carboxylic acid and 2,4-difluoroaniline.
m.p. 203-205°C

### Example 13

### Preparation of N-[1-methyl-4-(3-methoxyphenyl)-1,2-dihydro-2-oxo-1,8-naphthyridin-3-yl]-N'-(2,4,6-trimethylphenyl)urea

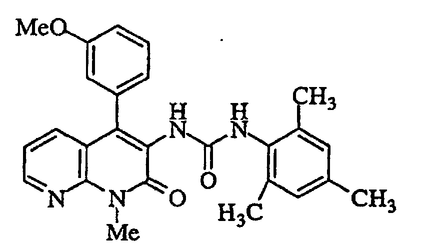

The title compound was obtained in the same manner as in Example 7 from 1-methyl-4-(3-methoxyphenyl)-1,2-dihydro-2-oxo-1,8-naphthyridine-3-carboxylic acid and 2,4,6-trimethylaniline.
m.p. >230°C
IR (KBr) 2956, 1654, 1585, 1547, 1460, 1254 cm⁻¹

### Example 14

### Preparation of N-[1-methyl-4-(3-methoxyphenyl)-1,2-dihydro-2-oxo-1,8-naphthyridin-3-yl]-N'-(2-trifluoromethylphenyl)urea

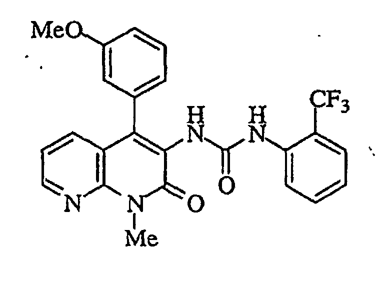

The title compound was obtained in the same manner as in Example 7 from 1-methyl-4-(3-methoxyphenyl)-1,2-dihydro-2-oxo-1,8-naphthyridine-3-carboxylic acid and 2-trifluoromethylaniline.
¹H-NMR δ (CDCl₃) 8.60 (1H, dd, J=4.6Hz, 2.0Hz), 7.78 (1H, dd, J=8.3Hz, J=2.0Hz), 7.70 (1H, d, J=8.3Hz), 7.32-7.54 (4H, m), 7.12-7.17 (3H, m), 6.90-6.98 (3H, m), 3.85 (3H, s), 3.80 (3H, s)

### Example 15

### Preparation of N-[1-methyl-4-(3-methoxyphenyl)-1,2-dihydro-2-oxo-1,8-naphthyridin-3-yl]-N'-(2,6-dichlorophenyl)urea

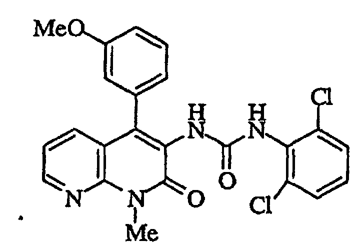

The title compound was obtained in the same manner as in Example 7 from 1-methyl-4-(3-methoxyphenyl)-1,2-dihydro-2-oxo-1,8-naphthyridine-3-carboxylic acid and 2,6-dichloroaniline.
m.p. 207-208°C

### Example 16

### Preparation of N-[1-methyl-4-(3-methoxyphenyl)-1,2-dihydro-2-oxo-1,8-naphthyridin-3-yl]-N'-(2-chloro-3-pyridyl)urea

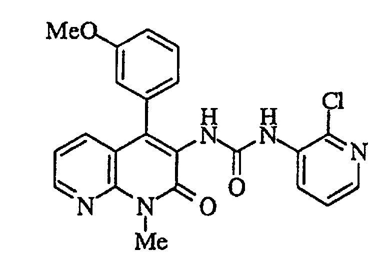

The title compound was obtained in the same manner as in Example 7 from 1-methyl-4-(3-methoxyphenyl)-1,2-dihydro-2-oxo-1,8-naphthyridine-3-carboxylic acid and 3-amino-2-chloropyridine.
m.p. >230°C
IR (KBr) 1652, 1587, 1535, 459, 1390, 1260 cm⁻¹

### Example 17

### Preparation of N-[1-methyl-4-(3-methoxyphenyl)-1,2-dihydro-2-oxo-1,8-naphthyridin-3-yl]-N'-(3,5-dichloro-2-pyridyl)urea

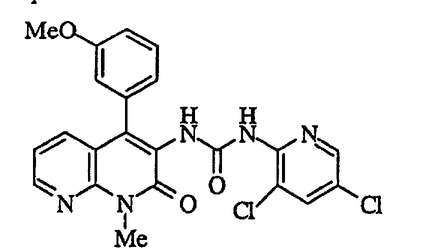

The title compound was obtained in the same manner as in Example 7 from 1-methyl-4-(3-methoxyphenyl)-1,2-dihydro-2-oxo-1,8-naphthyridine-3-carboxylic acid and 2-amino-3,5-dichloropyridine.
m.p. 78-79°C

### Example 18

### Preparation of N-[1-methyl-4-(3-methoxyphenyl)-1,2-dihydro-2-oxo-1,8-naphthyridin-3-yl]-N'-(8-quinolyl)urea

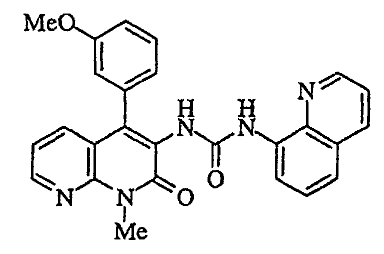

The title compound was obtained in the same manner as in Example 7 from 1-methyl-4-(3-methoxyphenyl)-1,2-dihydro-2-oxo-1,8-naphthyridine-3-carboxylic acid and 8-aminoquinoline.
m.p. >250°C
IR (KBr) 2924, 1710,1661, 1641, 1545 cm⁻¹

### Example 19

### Preparation of N-[1-methyl-4-(3-methoxyphenyl)-1,2-dihydro-2-oxo-1,8-naphthyridin-3-yl]-N'-(2-methoxy-4-nitrophenyl)urea

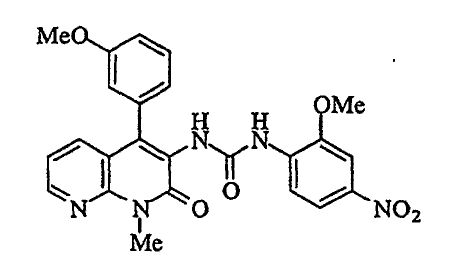

The title compound was obtained in the same manner as in Example 7 from 1-methyl-4-(3-methoxyphenyl)-1,2-dihydro-2-oxo-1,8-naphthyridine-3-carboxylic acid and 2-methoxy-4-nitroaniline.
m.p. >250°C
IR (KBr) 1715, 1664, 1645, 1588, 1550 cm⁻¹

### Example 20

### Preparation of N-[1-methyl-4-(3-methoxyphenyl)-1,2-dihydro-2-oxo-1,8-naphthyridin-3-yl]-N'-(2-isopropylphenyl)urea

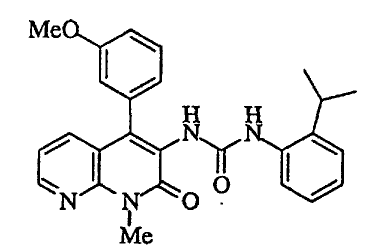

The title compound was obtained in the same manner as in Example 7 from 1-methyl-4-(3-methoxyphenyl)-1,2-dihydro-2-oxo-1,8-naphthyridine-3-carboxylic acid and 2-isopropylaniline.
m.p. 208-209°C

### Example 21

### Preparation of N-[1-methyl-4-(3-methoxyphenyl)-1,2-dihydro-2-oxo-1,8-naphthyridin-3-yl]-N'-(2-ethylphenyl)urea

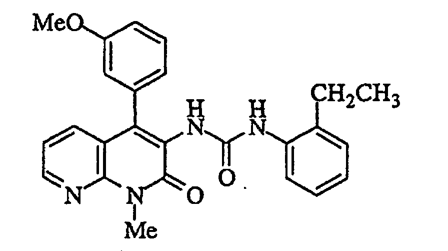

The title compound was obtained in the same manner as in Example 7 from 1-methyl-4-(3-methoxyphenyl)-1,2-dihydro-2-oxo-1,8-naphthyridine-3-carboxylic acid and 2-ethylaniline.
m.p. 212-212.5°C

### Example 22

### Preparation of N-[1-methyl-4-(3-methoxyphenyl)-1,2-dihydro-2-oxo-1,8-naphthyridin-3-yl]-N'-(2-isopropyl-6-methylphenyl)urea

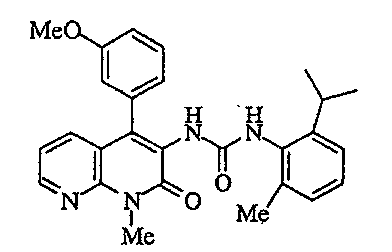

The title compound was obtained in the same manner as in Example 7 from 1-methyl-4-(3-methoxyphenyl)-1,2-dihydro-2-oxo-1,8-naphthyridine-3-carboxylic acid and 2-isopropyl-6-methylaniline.
m.p. 196-198°C

### Example 23

### Preparation of N-[1-methyl-4-(3-methoxyphenyl)-1,2-dihydro-2-oxo-1,8-naphthyridin-3-yl]-N'-butylurea

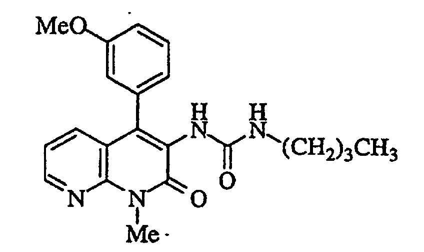

The title compound was obtained in the same manner as in Example 1 from 1-methyl-4-(3-methoxyphenyl)-1,2-dihydro-2-oxo-1,8-naphthyridine-3-carboxylic acid and butylamine.
m.p. 208-210°C

### Example 24

### Preparation of N-[1-methyl-4-(2-methoxyphenyl)-1,2-dihydro-2-oxo-1,8-naphthyridin-3-yl]-N'-(2,6-diisopropylphenyl)urea

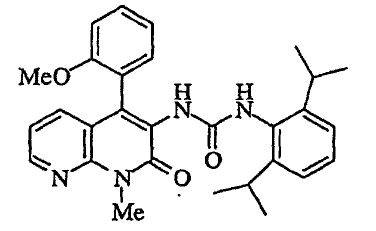

The title compound was obtained in the same manner as in Example 5 from 1-methyl-4-(2-methoxyphenyl)-1,2-dihydro-2-oxo-1,8-naphthyridine-3-carboxylic acid and 2,6-diisopropylaniline.
m.p. 179-180°C

### Example 25

### Preparation of N-[1-butyl-4-(3-methoxyphenyl)-1,2-dihydro-2-oxo-1,8-naphthyridin-3-yl]-N'-(2,6-diisopropylphenyl)urea

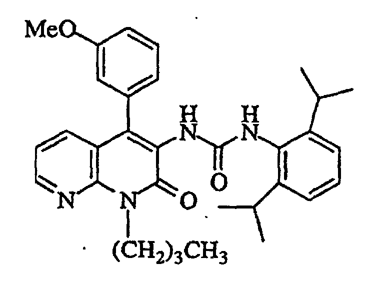

The title compound was obtained in the same manner as in Example 5 from 1-butyl-4-(3-methoxyphenyl)-1,2-dihydro-2-oxo-1,8-naphthyridine-3-carboxylic acid and 2,6-diisopropylaniline.
m.p. 179-182°C

### Example 26

### Preparation of N-[1-pentyl-4-(3-methoxyphenyl)-1,2-dihydro-2-oxo-1,8-naphthyridin-3-yl]-N'-(2,6-diisopropylphenyl)urea

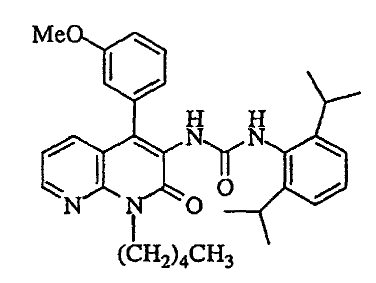

The title compound was obtained in the same manner as in Example 5 from 1-pentyl-4-(3-methoxyphenyl)-1,2-dihydro-2-oxo-1,8-naphthyridine-3-carboxylic acid and 2,6-diisopropylaniline.
m.p. 189-190°C

### Example 27

### Preparation of N-[1-(3-methylbutyl)-4-(3-methoxyphenyl)-1,2-dihydro-2-oxo-1,8-naphthyridin-3-yl]-N'-(2,6-diisopropylphenyl)urea

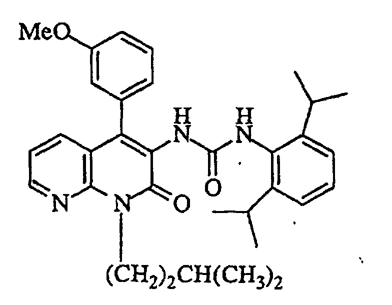

The title compound was obtained in the same manner as in Example 5 from 1-(3-methylbutyl)-4-(3-methoxyphenyl)-1,2-dihydro-2-oxo-1,8-naphthyridine-3-carboxylic acid and 2,6-diisopropylaniline.
m.p. 189.5-191°C

### Example 28

### Preparation of N-[1-butyl-4-(3-pyridyl)-1,2-dihydro-2-oxo-1,8-naphthyridin-3-yl]-N'-(2,6-diisopropylphenyl)urea

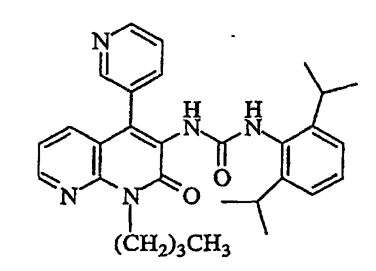

The title compound was obtained in the same manner as in Example 5 from 1-butyl-4-(3-pyridyl)-1,2-dihydro-2-oxo-1,8-naphthyridine-3-carboxylic acid and 2,6-diisopropylaniline.
Hydrochloride: m.p. 170-172°C

### Example 29

### Preparation of N-[1-(3-cyanopropyl)-4-(3-methoxyphenyl)-1,2-dihydro-2-oxo-1,8-naphthyridin-3-yl]-N'-(2,6-diisopropylphenyl)urea

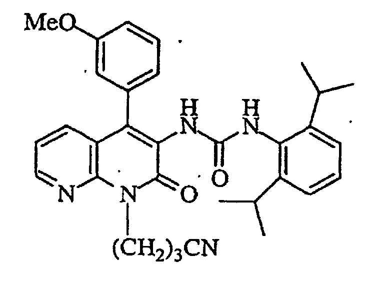

The title compound was obtained in the same manner as in Example 5 from 1-(3-cyanopropyl)-4-(3-methoxyphenyl)-1,2-dihydro-2-oxo-1,8-naphthyridine-3-carboxylic acid and 2,6-diisopropylaniline.
m.p. 187-188.5°C

### Example 30

### Preparation of N-(1-methyl-4-phenyl-1,2-dihydro-2-oxo-1,8-naphthyridin-3-yl)-N'-(2,6-diisopropylphenyl)urea

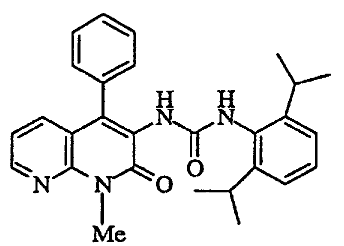

To a solution of N-[1-methyl-4-(2-chlorophenyl)-1,2-dihydro-2-oxo-1,8-naphthyridin-3-yl]-N'-(2,6-diisopropylphenyl)urea (350 mg, 0.72 mmol) in methanol (20 ml) were added ammonium formate (135 mg, 2.15 mmol) and 10 % palladium-carbon (100 mg), and the mixture was refluxed for 4 hours. After allowed to stand for cooling, the mixture was filtered through a cerite pad, and the filtrate was concentrated under reduced pressure. To the residue was added dilute aqueous ammonia, and the mixture was extracted with ethyl acetate. The extract was washed with water, washed with a saturated sodium chloride solution, dried over anhydrous magnesium sulfate, concentrated under reduced pressure, and crystallized from diethyl ether to give the title compound (249 mg, mmol) as a colorless powder.
m.p. 188-190.5°C

### Example 31

### Preparation of N-(1-methyl-4-(3-hydroxyphenyl)-1,2-dihydro-2-oxo-1,8-naphthyridin-3-yl)-N'-(2,6-diisopropylphenyl)urea

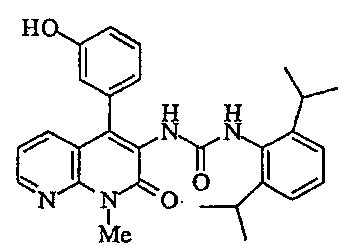

To a solution of N-[1-methyl-4-(3-methoxyphenyl)-1,2-dihydro-2-oxo-1,8-naphthyridin-3-yl]-N'-(2,6-diisopropylphenyl)urea (1310 mg, 2.7 mmol) in methylene chloride (20 ml) was added dropwise boron tribromide (1.7 g, 6.75 mmol) at 0°C, and the mixture was stirred for 6 hours. The mixture was poured into a saturated aqueous sodium hydrogen carbonate solution, and the mixture was extracted with methylene chloride. The extract was washed with water, washed with a saturated sodium chloride solution, and dried over anhydrous magnesium sulfate. The residue was concentrated under reduced pressure, purified by silica gel column chromatography (3 % methanol in chloroform), and crystallized from diethyl ether/hexane to give the title compound (830 mg, 1.76 mmol) as a colorless powder.
m.p. 152-155°C

### Example 32

### Preparation of N-(1-butyl-4-(3-hydroxyphenyl)-1,2-dihydro-2-oxo-1,8-naphthyridin-3-yl)-N'-(2,6-diisopropylphenyl)urea

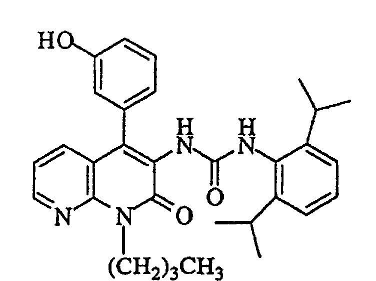

The title compound was obtained in the same manner as in Example 31 from N-[1-butyl-4-(3-methoxyphenyl)-1,2-dihydro-2-oxo-1,8-naphthyridin-3-yl]-N'-(2,6-diisopropylphenyl)urea.
m.p. 136-140°C

### Example 33

### Preparation of N-(1-methyl-4-(3-hydroxyphenyl)-1,2-dihydro-2-oxo-1,8-naphthyridin-3-yl)-N'-butylurea

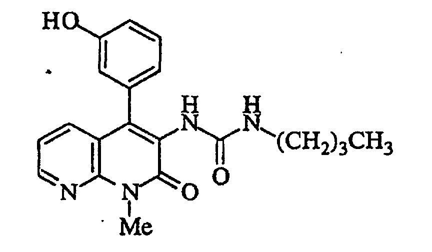

The title compound was obtained in the same manner as in Example 31 from N-[1-methyl-4-(3-methoxyphenyl)-1,2-dihydro-2-oxo-1,8-naphthyridin-3-yl]-N'-butylurea.
m.p. 178-180.5°C

### Example 34

### Preparation of N-[1-butyl-4-{3-(2-acetoxyethoxy)phenyl}-1,2-dihydro-2-oxo-1,8-naphthyridin-3-yl]-N'-(2,6-diisopropylphenyl)urea

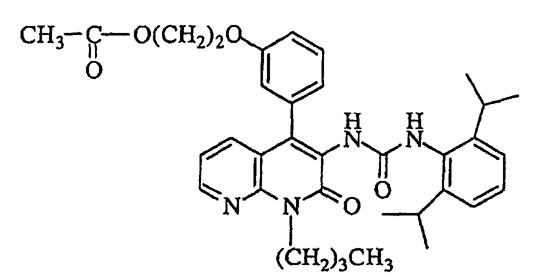

To a solution of N-(1-butyl-4-(3-hydroxyphenyl)-1,2-dihydro-2-oxo-1,8-naphthyridin-3-yl)-N'-(2,6-diisopropylphenyl)urea (300 mg, 0.59 mmol) in DMF (10 ml) was added sodium hydride (NaH, 23 mg, 0.59 mmol), and the mixture was stirred at room temperature for 0.5 hour. To the mixture was added 2-bromoethyl acetate (98 mg, 0.59 mmol), and the mixture was stirred at 40-50°C for 6 hours. After allowed to stand for cooling, the mixture was poured into water, extracted with ethyl acetate, and the extract was washed with water, washed with a saturated sodium chloride solution, and dried over anhydrous magnesium sulfate. The residue was concentrated under reduced pressure, and purified by silica gel column chromatography (3 % methanol in chloroform) to give the title compound (209 mg, 0.35 mmol) as a colorless powder.
¹H-NMR δ (CDCl₃) 8.53 (1H, br), 7.65 (1H, br), 6.98-7.44 (9H, m), 6.27 (0.5H, br), 5.72 (0.5H, br), 4.53 (2H, br), 4.44 (2H, m), 4.20 (2H, m), 3.06 (2H, br), 2.11 (3H, s), 1.72 (2H, br), 1.45 (2H, m), 1.11-1.24 (12H, m), 0.96 (3H, t, J=7.3Hz)

### Example 35

### Preparation of N-[1-methyl-4-[3-{3-(4-phenyl-1-piperazinyl)propoxy}phenyl]-1,2-dihydro-2-oxo-1,8-naphthyridin-3-yl]-N'-(2,6-diisopropylphenyl)-urea

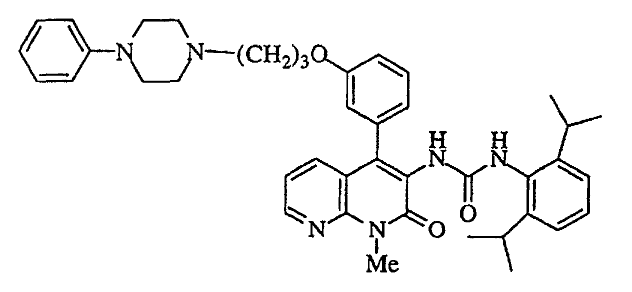

The title compound was obtained in the same manner as in Example 34 from N-{1-methyl-4-(3-hydroxyphenyl)-1,2-dihydro-2-oxo-1,8-naphthyridin-3-yl}-N'-(2,6-diisopropylphenyl)urea and 1-(3-chloropropyl)-4-phenylpiperazine.
¹H-NMR δ (CDCl₃) 8.54 (1H, m), 7.65 (1H, m), 6.83-7.42 (14H, m), 6.10 (0.5H, br), 5.77 (1H, br), 4.09 (2H, t, J=6.3Hz), 3.87 (3H, br), 3.22 (4H, br), 2.51-3.10 (2H, br), 2.64 (6H, br), 2.05 (2H, t, J=6.3Hz), 1.11-1.25 (12H, br)

### Example 36

### Preparation of N-[1-methyl-4-{3-(2-acetoxyethoxy)phenyl}-1,2-dihydro-2-oxo-1,8-naphthyridin-3-yl]-N'-(2,6-diisopropylphenyl)urea

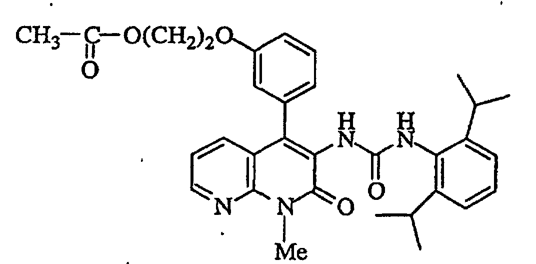

The title compound was obtained in the same manner as in Example 34 from N-{1-methyl-4-(3-hydroxyphenyl)-1,2-dihydro-2-oxo-1,8-naphthyridin-3-yl}-N'-(2,6-diisopropylphenyl)urea and 2-bromoethyl acetate.
¹H-NMR δ (CDCl₃) 8.53 (1H, m), 7.62-7.74 (1H, m), 6.87-7.40 (9H, m), 6.13 (0.5H, br), 5.93 (0.5H, br), 4.43 (2H, m), 4.20 (2H, m), 3.93 (3H, br), 2.94-3.21 (2H, br), 2.11 (3H, s), 1.08-1.26 (12H, br)

### Example 37

### Preparation of N-[1-butyl-4-{3-(3-phthalimidopropoxy)phenyl}-1,2-dihydro-2-oxo-1,8-naphthyridin-3-yl]-N'-(2,6-diisopropylphenyl)urea

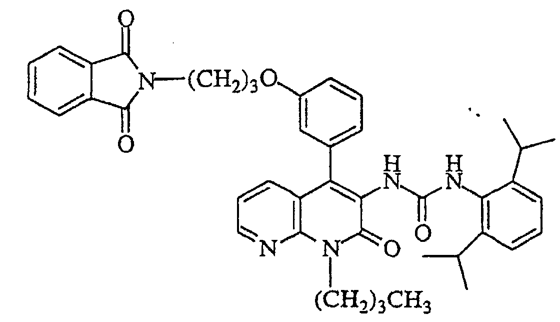

The title compound was obtained in the same manner as in Example 34 from N-{1-butyl-4-(3-hydroxyphenyl)-1,2-dihydro-2-oxo-1,8-naphthyridin-3-yl}-N'-(2,6-diisopropylphenyl)urea and N-(3-bromopropyl)phthalimide.
¹H-NMR δ (CDCl₃) 8.52 (1H, br), 7.79-7.82 (2H, m), 7.64-7.68 (3H, m), 6.82-7.36 (9H, m), 6.21 (0.5H, br), 5.78 (0.5H, br), 4.54 (2H, br), 4.07 (2H, t, J=5.9Hz), 3.91 (2H, t, J=6.9Hz), 2.95-3.20 (2H, br), 2.23 (2H, m), 1.73 (2H, m), 1.56 (2H, m), 1.09-1.9 (12H, m), 0.97 (3H, brt, J=7.3Hz)

### Example 38 (not encompassed by the present invention)

### Preparation of N-[1-methyl-4-[3-{3-(4-phenyl-1-piperazinyl)propoxy}phenyl]-1,2-dihydro-2-oxo-1,8-naphthyridin-3-yl]-N'-butylurea

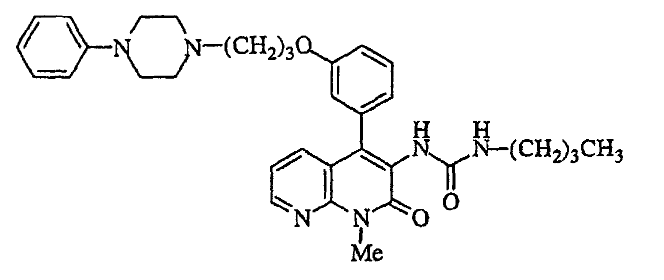

The title compound was obtained in the same manner as in Example 34 from N-{1-methyl-4-(3-hydroxyphenyl)-1,2-dihydro-2-oxo-1,8-naphthyridin-3-yl}-N'-butylurea and 1-(3-chloropropyl)-4-phenylpiperazine.
m.p. 120.5-121.5°C

### Example 39

### Preparation of N-[1-butyl-4-{3-(3-chloropropoxy)phenyl}-1,2-dihydro-2-oxo-1,8-naphthyridin-3-yl]-N'-(2,6-diisopropylphenyl)urea

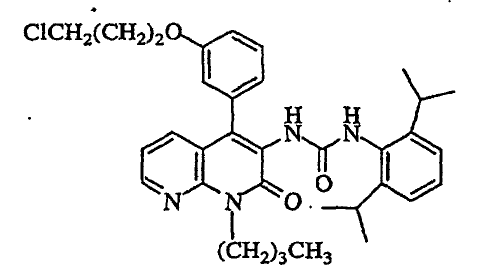

To a solution of N-{1-butyl-4-(3-hydroxyphenyl)-1,2-dihydro-2-oxo-1,8-naphthyridin-3-yl}-N'-(2,6-diisopropylphenyl)urea (1000 mg, 1.95 mmol) and 1-bromo-3-chloropropane (460 mg, 2.93 mmol) in DMF (10 ml) was added potassium carbonate (673 mg, 4.88 mmol), and the mixture was stirred at 50-60°C for 6 hours. After allowed to stand for cooling, the mixture was poured into water, extracted with ethyl acetate, washed with water, washed with a saturated aqueous sodium chloride solution, and dried over anhydrous sodium sulfate. The residue was concentrated under reduced pressure, purified by silica gel column chromatography (ethyl acetate:hexane = 50:50), and crystallized from hexane to give the title compound (1020 mg, 1.73 mmol) as a colorless powder.
m.p. 138-140°C

### Example 40

### Preparation of N-[1-(3-cyanopropyl)-4-{3-(3-chloropropoxy)phenyl}-1,2-dihydro-2-oxo-1,8-naphthyridin-3-yl]-N'-(2,6-diisopropylphenyl)urea

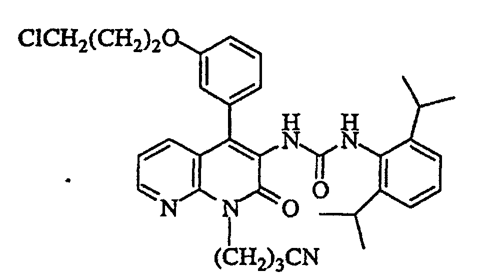

The title compound was obtained in the same manner as in Example 39 from N-{1-(3-cyanopropyl)-4-(3-hydroxyphenyl)-1,2-dihydro-2-oxo-1,8-naphthyridin-3-yl}-N'-(2,6-diisopropylphenyl)urea and 1-bromo-3-chloropropane.
¹H-NMR δ (CD₃OD) 8.60 (1H, d, J=4.6Hz), 7.72 (1H, d, J=6.3Hz), 7.45 (1H, dd, J=7.9Hz, 7.9Hz), 7.07-7.25 (5H, m), 6.96 (2H, br), 4.78 (2H, t, J=6.6Hz), 4.16 (2H, t, J=6.6Hz), 3.75 (2H, t, J=6.6Hz), 2.95-3.05 (2H, m), 2.60 (2H, t, J=7.2Hz)

### Example 41

### Preparation of N-[1-butyl-4-{3-(3-dimethylaminopropoxy)phenyl}-1,2-dihydro-2-oxo-1,8-naphthyridin-3-yl]-N'-(2,6-diisopropylphenyl)urea

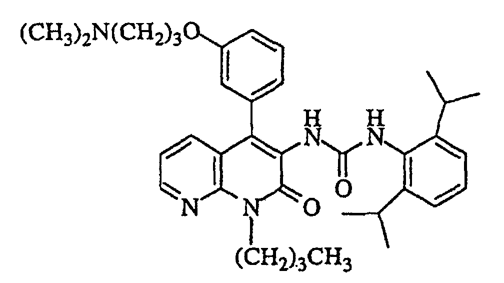

To a suspension of N-{1-butyl-4-(3-hydroxyphenyl)-1,2-dihydro-2-oxo-1,8-naphthyridin-3-yl}-N'-(2,6-diisopropylphenyl)urea (200 mg, 0.4 mmol), potassium carbonate (166 mg, 1.2 mmol), and sodium iodide (5 mg) in DMF (10 ml) was added 3-dimethylaminopropyl chloride hydrochloride (63 mg) at room temperature, and the mixture was stirred at 60-70°C for 10 hours. After allowed to stand for cooling, the mixture was poured into water, extracted with ethyl acetate, washed with water, washed with a saturated aqueous sodium chloride solution, and dried over anhydrous magnesium sulfate. The residue was concentrated under reduced pressure, and purified by silica gel column chromatography (10 % methanol in chloroform) to give the title compound (88 mg, 0.15 mmol).
¹H-NMR δ (DMSO-d₆) 8.59 (1H, d, J=3.3Hz), 7.76 (1H, s), 7.74 (1H, s), 7.61 (1H, d, J=6.6Hz), 7.38 (1H, dd, J=7.6Hz, 7.6Hz), 7.12-7.26 (2H, m), 6.98-7.04 (3H, m), 6.85-6.91 (2H, m), 4.52 (2H, br), 3.99 (2H, brt, J=6.9Hz), 2.85-2.95 (2H, m), 2.38 (2H, t, J=6.9Hz), 1.82-1.91 (2H, m), 1.65-1.75 (2H, m), 1.37-1.47 (2H, m), 0.95-1.00 (15H, m)

### Example 42 (not encompassed by the present invention)

### Preparation of N-[1-methyl-4-(3-benzyloxyphenyl)-1,2-dihydro-2-oxo-1,8-naphthyridin-3-yl]-N'-butylurea

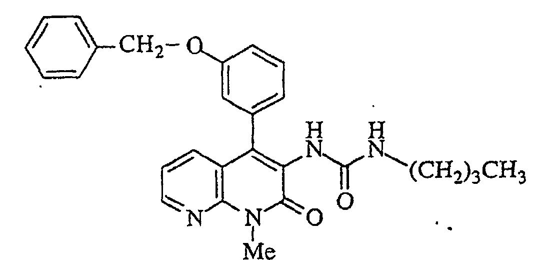

The title compound was obtained in the same manner as in Example 41 from N-[1-methyl-4-(3-hydroxyphenyl)-1,2-dihydro-2-oxo-1,8-naphthyridin-3-yl]-N'-butylurea and benzyl bromide.
m.p. 183-184°C

### Example 43

### Preparation of N-[1-butyl-4-{3-(4-pyridylmethoxy)phenyl}-1,2-dihydro-2-oxo-1,8-naphthyridin-3-yl]-N'-(2,6-diisopropylphenyl)urea

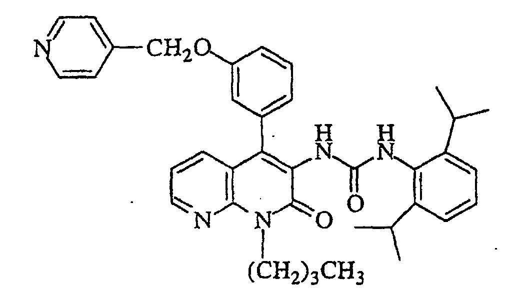

The title compound was obtained in the same manner as in Example 41 from N-{1-butyl-4-(3-hydroxyphenyl)-1,2-dihydro-2-oxo-1,8-naphthyridin-3-yl}-N'-(2,b-diisopropylphenyl)urea and 4-picolyl chloride hydrochloride.
m.p. 157-158°C

### Example 44

### Preparation of N-[1-butyl-4-{3-(2-pyridylmethoxy)phenyl}-1,2-dihydro-2-oxo-1,8-naphthyridin-3-yl]-N'-(2,6-diisopropylphenyl)urea

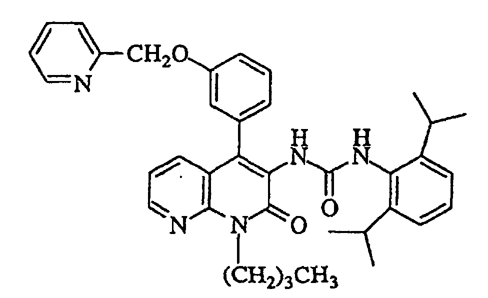

The title compound was obtained in the same manner as in Example 41 from N-{1-butyl-4-(3-hydroxyphenyl)-1,2-dihydro-2-oxo-1,8-naphthyridin-3-yl}-N'-(2,6-diisopropylphenyl)urea and 2-picolyl chloride hydrochloride.

Hydrochloride: m.p. 145-146°C

### Example 45

### Preparation of N-[1-butyl-4-{3-(3-piperidinopropoxy)phenyl}-1,2-dihydro-2-oxo-1,8-naphthyridin-3-yl]-N'-(2,6-diisopropylphenyl)urea

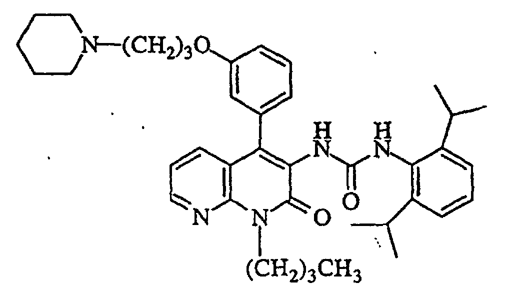

The title compound was obtained in the same manner as in Example 41 from N-{1-butyl-4-(3-hydroxyphenyl)-1,2-dihydro-2-oxo-1,8-naphthyridin-3-yl}-N'-(2,6-diisopropylphenyl)urea and N-(3-chloropropyl)piperidine hydrochloride.
Hydrochloride: m.p. 142-145°C

### Example 46

### Preparation of N-[1-butyl-4-{3-(2-diethylaminoethoxy)phenyl}-1,2-dihydro-2-oxo-1,8-naphthyridin-3-yl]-N'-(2,6-diisopropylphenyl)urea

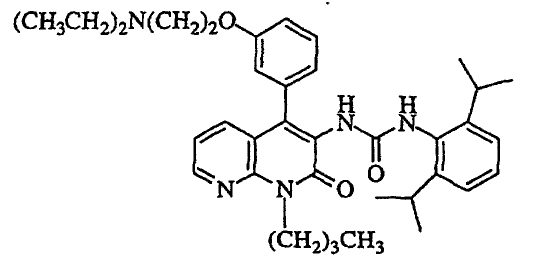

The title compound was obtained in the same manner as in Example 41 from N-{1-butyl-4-(3-hydroxyphenyl)-1,2-dihydro-2-oxo-1,8-naphthyridin-3-yl}-N'-(2,6-diisopropylphenyl)urea and 2-diethylaminoethyl chloride hydrochloride.
Hydrochloride: m.p. 141-144°C

### Example 47

### Preparation of N-[1-butyl-4-{3-(2-dimethylaminoethoxy)phenyl}-1,2-dihydro-2-oxo-1,8-naphthyridin-3-yl]-N'-(2,6-diisopropylphenyl)urea

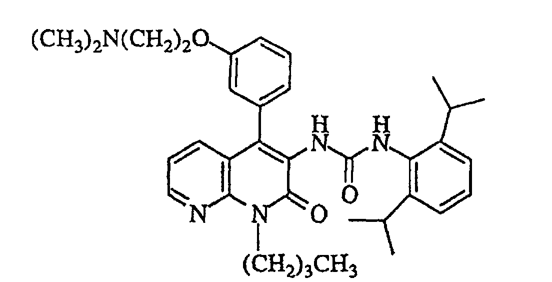

The title compound was obtained in the same manner as in Example 41 from N-{1-butyl-4-(3-hydroxyphenyl)-1,2-dihydro-2-oxo-1,8-naphthyridin-3-yl}-N'-(2,6-diisopropylphenyl)urea and 2-dimethylaminoethyl chloride hydrochloride.
¹H-NMR δ (CD₃OD) 8.59 (1H, d, J=4.6Hz), 7.72 (1H, d, J=6.3Hz), 7.44 (1H, dd, J=8.3H, 8.3Hz), 7.15-7.22 (3H, m), 7.07 (2H, d, J=7.3Hz), 6.98 (2H, m), 4.64 (2H, t, J=7.6Hz), 4.15 (2H, t, J=5.0Hz), 2.96 (2H, sep, J=6.9Hz), 2.83 (2H, t, J=5.0Hz), 1.70-1.81 (2H, m), 1.40-1.55 (2H, m), 1.10 (12H, d, J=6.9Hz), 1.02 (3H, t, J=7.3Hz)

### Example 48

### Preparation of N-[1-butyl-4-{3-(3-benzyloxypropoxy)phenyl}-1,2-dihydro-2-oxo-1,8-naphthyridin-3-yl]-N'-(2,6-diisopropylphenyl)urea

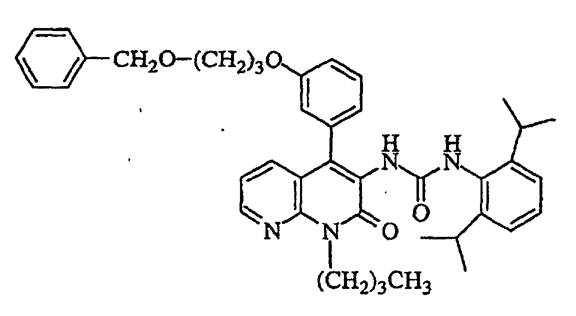

The title compound was obtained in the same manner as in Example 41 from N-{1-butyl-4-(3-hydroxyphenyl)-1,2-dihydro-2-oxo-1,8-naphthyridin-3-yl}-N'-(2,6-diisopropylphenyl)urea and benzyl 3-bromopropyl ether.
¹H-NMR δ (CD₃OD) 8.58 (1H, br), 7.72 (1H, d, J=8.3Hz), 7.39 (1H, t, J=7.9Hz), 6.94-7.29 (12H, m), 4.63 (2H, t, J=7.6Hz), 4.12 (2H, t, J=5.9Hz), 3.66 (2H, t, J=5.9Hz), 2.90-3.00 (2H, m), 2.00-2.10 (2H, m), 1.73-1.85 (2H, m), 1.48-1.56 (2H, m), 1.10 (12H, d, J=6.9Hz), 1.02 (3H, t, J=7.3Hz)

### Example 49

### Preparation of N-[1-(3-cyanopropyl)-4-{3-(2-acetoxyethoxy)phenyl}-1,2-dihydro-2-oxo-1,8-naphthyridin-3-yl]-N'-(2,6-diisopropylphenyl)urea

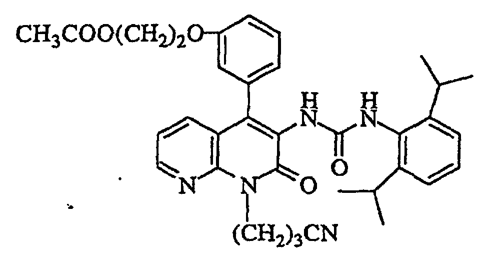

The title compound was obtained in the same manner as in Example 41 from N-{1-(3-cyanopropyl)-4-(3-hydroxyphenyl)-1,2-dihydro-2-oxo-1,8-naphthyridin-3-yl}-N'-(2,6-diisopropylphenyl)urea and 2-bromoethyl acetate.
¹H-NMR δ (CD₃OD) 8.60 (1H, d, J=2.6Hz), 7.72 (1H, d, J=6.3Hz), 7.46 (1H, dd, J=7.9Hz, 7.9Hz), 7.07-7.25 (5H, m), 6.98 (2H, br), 4.78 (2H, t, J=6.9Hz), 4.38 (2H, t, J=5.0Hz), 4.22 (2H, t, J=5.0Hz), 2.86-3.00 (2H, m), 2.60 (2H, t, J=7.3Hz), 2.10-2.22 (2H, m), 2.05 (3H, s), 1.11 (12H, brd, J=5.9Hz)

### Example 50

Preparation of N-[1-(3-cyanopropyl)-4-{3-(2-diethylaminoethoxy)-phenyl}-1,2-dihydro-2-oxo-1,8-naphthyridin-3-yl]-N'-(2,6-diisopropylphenyl)-urea

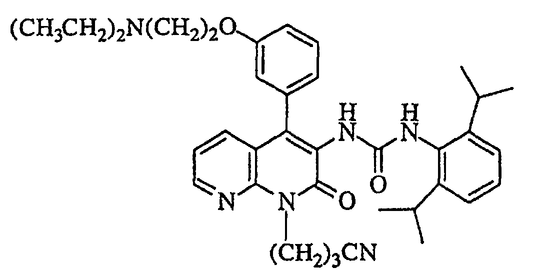

The title compound was obtained in the same manner as in Example 41 from N-{1-(3-cyanopropyl)-4-(3-hydroxyphenyl)-1,2-dihydro-2-oxo-1,8-naphthyridin-3-yl}-N'-(2,6-diisopropylphenyl)urea and 2-diethylaminoethyl chloride hydrochloride.
¹H-NMR δ (CD₃OD) 8.58 (1H, dd, J=4.6Hz, 1.6Hz), 7.70 (1H, dd, J=7.9Hz, 1.6Hz), 7.45 (1H, dd, J=7.9Hz, 7.9Hz), 7.06-7.22 (5H, m), 6.96 (2H, br), 4.75 (2H, t, J=6.9Hz), 4.14 (2H, t, J=4Hz), 2.92-3.04 (4H, m), 2.69 (4H, q, J=6.9Hz), 2.57 (2H, t, J=6.9Hz), 2.12-2.02 (2H, m), 1.06-1.12 (18H, m)

### Example 51

### Preparation of N-[1-butyl-4-{3-(2-hydroxyethoxy)phenyl}-1,2-dihydro-2-oxo-1,8-naphthyridin-3-yl]-N'-(2,6-diisopropylphenyl)urea

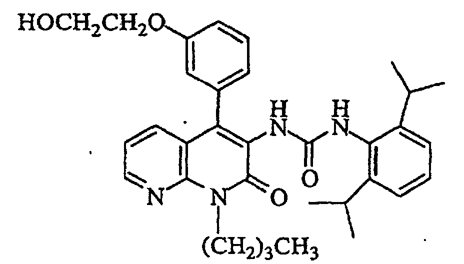

To a solution of N-[1-butyl-4-{3-(2-acetoxyethoxy)phenyl}-1,2-dihydro-2-oxo-1,8-naphthyridin-3-yl]-N'-(2,6-diisopropylphenyl)urea (196 mg, 0.33 mmol) in methanol (10 ml) was added potassium carbonate (11 mg), and the mixture was stirred at room temperature for 4 hours. To the mixture was added water, and the mixture was extracted with ethyl acetate. The extract was washed with water, washed with a saturated aqueous sodium chloride solution, and dried over magnesium sulfate. The residue was concentrated under reduced pressure, and crystallized from ether-hexane to give the title compound (145 mg, 0.26 mmol).
m.p. 106-110°C

### Example 52

### Preparation of N-[1-methyl-4-{3-(2-hydroxyethoxy)phenyl}-1,2-dihydro-2-oxo-1,8-naphthyridin-3-yl]-N'-(2,6-diisopropylphenyl)urea

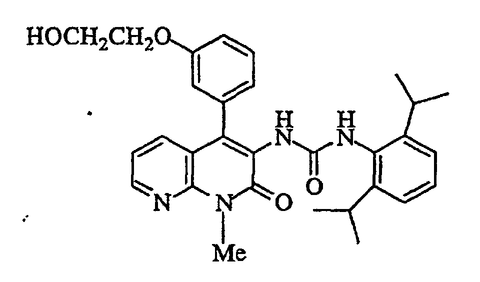

The title compound was obtained in the same manner as in Example 51 from N-[1-methyl-4-{3-(2-acetoxyethoxy)phenyl}-1,2-dihydro-2-oxo-1,8-naphthyridin-3-yl}-N'-(2,6-diisopropylphenyl)urea.
¹H-NMR δ (CDCl₃) 8.53 (1H, dd, J=3.6Hz), 7.42 (1H, m), 6.89-7.42 (9H, m), 6.11 (0.5H, br), 5.86 (0.5H, br), 4.13 (2H, br), 3.96 (3H, br), 3.87 (2H, br), 2.90-3.25 (2H, br), 1.09-1.29 (12H, m)

### Example 53

### Preparation of N-[1-(3-cyanopropyl)-4-{3-(2-hydroxyethoxy)phenyl}-1,2-dihydro-2-oxo-1,8-naphthyridin-3-yl]-N'-(2,6-diisopropylphenyl)urea

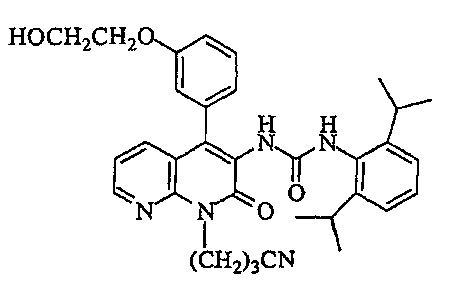

The title compound was obtained in the same manner as in Example 51 from N-[1-(3-cyanopropyl)-4-{3-(2-acetoxyethoxy)phenyl}-1,2-dihydro-2-oxo-1,8-naphthyridin-3-yl}-N'-(2,6-diisopropylphenyl)urea.
¹H-NMR δ (CD₃OD) 8.60 (1H, d, J=4.6Hz), 7.73 (1H, d, J=6.3Hz), 7.45 (1H, dd, J=8.3Hz, 8.3Hz), 7.07-7.25 (5H, m), 6.95-6.98 (2H, m), 4.78 (2H, t, J=6.9Hz), 4.08 (2H, t, J=4.9Hz), 3.87 (2H, t, J=4.9Hz), 2.93-3.04 (2H, m), 2.60 (2H, t, J=7.3Hz), 1.12 (12H, br)

### Example 54

### Preparation of N-[1-butyl-4-{3-(3-hydroxypropoxy)phenyl}-1,2-dihydro-2-oxo-1,8-naphthyridin-3-yl]-N'-(2,6-diisopropylphenyl)urea

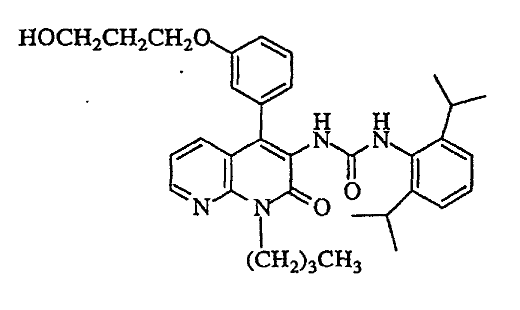

To a solution of N-[1-butyl-4-{3-(3-benzyloxypropoxy)phenyl}-1,2-dihydro-2-oxo-1,8-naphthyridin-3-yl]-N'-(2,6-diisopropylphenyl)urea (316 mg, 0.48 mmol) in methanol (20 ml) were added ammonium formate (91 mg, 1.44 mmol) and 10 % palladium-carbon (100 mg), and the mixture was refluxed for 10 hours. After allowed to stand for cooling, the mixture was filtered through a cerite pad, and the filtrate was concentrated under reduced pressure. The residue was dissolved in ethyl acetate, and the mixture was washed with water, washed with a saturated aqueous sodium chloride solution, and dried over anhydrous magnesium sulfate. The residue was concentrated under reduced pressure, purified by silica gel column chromatography (1-3 % methanol in chloroform), and crystallized from ether-hexane to give the title compound (85 mg, 0.15 mmol).
¹H-NMR δ (CD₃OD) 8.58 (1H, dr), 7.72 (1H, d, J=8.3Hz), 7.42 (1H, t, J=8.3Hz), 7.06-7.20 (5H, m), 6.95 (2H, br), 4.63 (2H, t, J=7.3Hz), 4.10 (2H, t, J=6.3Hz), 3.72 (2H, t, J=5.9Hz), 2.96 (2H, sep, J=6.6Hz), 1.99 (2H, t, J=6.3Hz), 1.78 (2H, br), 1.47 (2H, br), 1.10 (12H, d, J=6.6Hz), 1.02 (3H, t, J=7.3Hz)

### Example 55

### Preparation of N-[1-butyl-4-{3-(3-aminopropoxy)phenyl}-1,2-dihydro-2-oxo-1,8-naphthyridin-3-yl]-N'-(2,6-diisopropylphenyl)urea

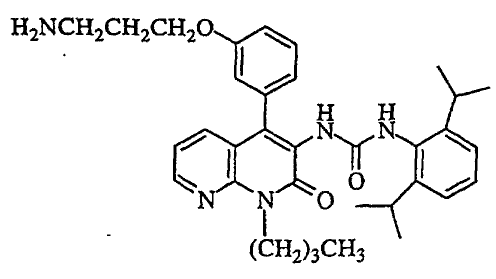

To a solution of N-[1-butyl-4-{3-(3-phthalimidopropoxy)phenyl}-1,2-dihydro-2-oxo-1,8-naphthyridin-3-yl]-N'-(2,6-diisopropylphenyl)urea (157 mg, 0.22 mmol) in ethanol (10 ml) was added hydrazine monohydrate (100 mg, 2 mmol), and the mixture was stirred at room temperature for 3 hours. The precipitates were removed by filtration, and the filtrate was concentrated under reduced pressure. To the residue was added methylene chloride, and the insoluble materials were removed by filtration, and the filtrate was concentrated under reduced pressure. The residue was purified by silica gel column chromatography (10 % methanol in chloroform) to give the title compound (58 mg, 0.10 mmol).
Hydrochloride: m.p. 106-110°C

### Example 56

### Preparation of N-[1-butyl-4-[3-{3-(1-imidazolyl)propoxy}phenyl]-1,2-dihydro-2-oxo-1,8-naphthyridin-3-yl]-N'-(2,6-diisopropylphenyl)urea

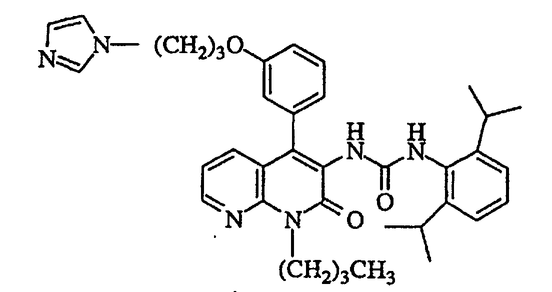

To a suspension of imidazole (97 mg, 1.43 mmol), potassium carbonate (246 mg, 1.78 mmol) and sodium iodide (35 mg) in DMF (20 ml) was added N-[1-(butyl-4-{3-(3-chloropropoxy)phenyl}-1,2-dihydro-2-oxo-1,8-naphthyridin-3-yl]-N'-(2,6-diisopropylphenyl)urea (700 mg, 1.19 mmol), and the mixture was stirred at 50-60°C for 10 hours. After allowed to stand for cooling, the mixture was poured into water, extracted with ethyl acetate, and the extract was washed with water, washed with a saturated aqueous sodium chloride solution, and dried over magnesium sulfate. The residue was concentrated under reduced pressure, and purified by silica gel column chromatography (5 % methanol in chloroform) to give the title compound (286 mg, 0.46 mmol).
¹H-NMR δ (DMSO-d₆) 8.60 (1H, d, J=4.6Hz), 7.76 (1H, s), 7.74 (1H, s), 7.61 (1H, d, J=7.0Hz), 7.60 (1H, s), 7.40 (1H, dd, J=7.9Hz, 7.9Hz), 7.12-7.26 (3H, m), 7.02-7.05 (3H, m), 6.86-6.93 (3H, m), 4.53 (2H, br), 4.08-4.14 (2H, m), 3.93 (2H, t, J=5.6Hz), 2.85-2.95 (2H, m), 2.20 (2H, br), 1.72 (2H, br), 1.39-1.47 (2H, m), 0.97-1.24 (15H, m)

### Example 57

### Preparation of N-[1-butyl-4-[3-{3-(1,2,4-triazol-1-yl)propoxy}phenyl]-1,2-dihydro-2-oxo-1,8-naphthyridin-3-yl]-N'-(2,6-diisopropylphenyl)urea

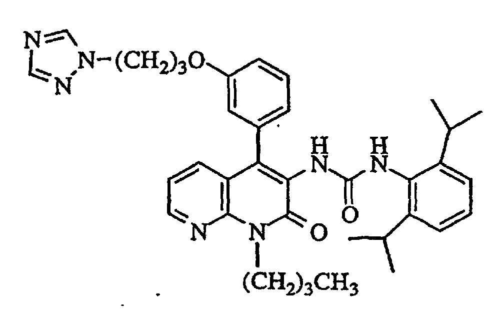

The title compound was obtained in the same manner as in Example 56 from N-[1-butyl-4-{3-(3-chloropropoxy)phenyl}-1,2-dihydro-2-oxo-1,8-naphthyridin-3-yl]-N'-(2,6-diisopropylphenyl)urea and 1,2,4-triazole.

¹H-NMR δ (CD₃OD) 8.60 (1H, d, J=3.0Hz), 8.43 (1H, s), 7.96 (1H, s), 7.71 (1H, d, J=7.9Hz), 7.43 (1H, dd, J=7.9Hz, 7.9Hz), 6.94-7.23 (7H, m), 4.64 (2H, brt, J=4.6Hz), 4.44 (2H, brt, J=4.4Hz), 4.03 (2H, br), 2.90-3.05 (2H, m), 2.37 (2H, m), 1.75-1.85 (2H, m), 1.45-1.56 (2H, m), 0.99-1.11 (15H, m)

### Example 58

### Preparation of N-[1-(3-phthalimidopropyl)-4-(3-methoxyphenyl)-1,2-dihydro-2-oxo-1,8-naphthyridin-3-yl]-N'-(2,6-diisopropylphenyl)urea

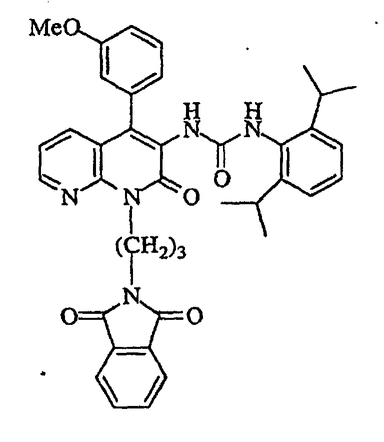

To a solution of N-[4-(3-methoxyphenyl)-1,2-dihydro-2-oxo-1,8-naphthyridin-3-yl]-N'-(2,6-diisopropylphenyl)urea (200 mg, 0.43 mmol) and N-(3-bromopropyl)phthalimide (133 mg, 0.50 mmol) in DMF (10 ml) was added potassium carbonate (114 mg, 0.83 mmol), and the mixture was stirred at 50-60°C for one hour. After allowed to stand for cooling, the mixture was poured into water, and the mixture was extracted with ethyl acetate. The extract was washed with water, washed with a saturated aqueous sodium chloride solution, and dried over anhydrous magnesium sulfate. The residue was concentrated under reduced pressure, purified by silica gel column chromatography (1-3 % methanol in chloroform), and crystallized from hexane to give the title compound (229 mg, 0.35 mmol).
¹H-NMR δ (CD₃OD) 8.40 (1H, dd, J=4.6Hz, 1.7Hz), 7.78-7.96 (4H, m), 7.72 (1H, dd, J=7.9Hz, 1.7Hz), 7.48 (1H, dd, J=8.2Hz, 7.9Hz), 7.04-7.27 (5H, m), 6.93-7.02 (2H, m), 4.66-4.78 (2H, m), 3.90 (2H, t, J=6.9Hz), 3.87 (3H, s), 3.02 (2H, sept, J=6.6Hz), 2.17-2.35 (2H, m), 1.13 (12H, brd, J=6.6Hz)

### Example 59

### Preparation of N-[1-(3-aminopropyl)-4-(3-methoxyphenyl)-1,2-dihydro-2-oxo-1,8-naphthyridin-3-yl]-N'-(2,6-diisopropylphenyl)urea

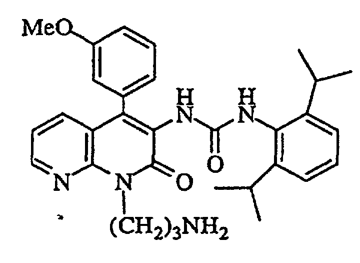

The title compound was obtained in the same manner as in Example 55 from N-[1-(phthalimidopropyl)-4-(3-methoxyphenyl)-1,2-dihydro-2-oxo-1,8-naphthyridin-3-yl}-N'-(2,6-diisopropylphenyl)urea.
¹H-NMR δ (CD₃OD) 8.40 (1H, dd, J=4.6Hz, 1.7Hz), 7.78-7.96 (4H, m), 7.72 (1H, dd, J=7.9Hz, 1.7Hz), 7.48 (1H, dd, J=8.2Hz, 7.9Hz), 7.04-7.27 (5H, m), 6.93-7.02 (2H, m), 4.66-4.78 (2H, m), 3.90 (2H, t, J=6.9Hz), 3.87 (3H, s), 3.02 (2H, sept, J=6.6Hz), 2.17-2.35 (2H, m), 1.13 (12H, brd, J=6.6Hz)

### Example 60

### Preparation of N-[1-(3-acetylaminopropyl)-4-(3-methoxyphenyl)-1,2-dihydro-2-oxo-1,8-naphthyridin-3-yl]-N'-(2,6-diisopropylphenyl)urea

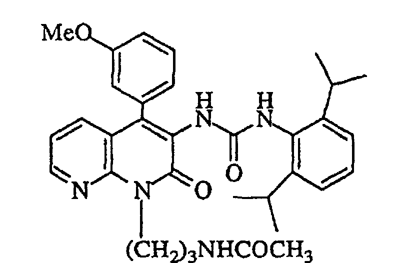

N-[1-(3-Aminoproyl)-4-(3-methoxyphenyl)-1,2-dihydro-2-oxo-1,8-naphthyridin-3-yl]-N'-(2,6-diisopropylphenyl)urea (50 mg, 0.1 mmol) was dissolved in pyridine (1 ml), and thereto was added acetic anhydride (0.5 ml). The mixture was stirred for 40 minutes, and concentrated under reduced pressure. The residue was purified by preparative thin layer chromatography to give the title compound (49 mg, 0.09 mmol).
¹H-NMR δ (CD₃OD) 8.65 (1H, dd, J=4.6H, 1.7Hz), 7.77 (1H, dd, J=7.9H, 1.7Hz), 7.50 (1H, dd, J=8.2Hz, 8.2Hz), 7.05-7.36 (5H, m), 6.95-7.03 (2H, m), 4.78 (2H, t, J=6.6Hz), 3.88 (3H, s), 3.04 (2H, sept, J=6.6Hz), 2.79 (2H, t, J=6.9Hz), 2.00-2.18 (2H, m), 1.15 (12H, brd, J=6.6Hz)

### Example 61

### Preparation of N-[1-(3-benzyloxypropyl)-4-(3-methoxyphenyl)-1,2-dihydro-2-oxo-1,8-naphthyridin-3-yl]-N'-(2,6-diisopropylphenyl)urea

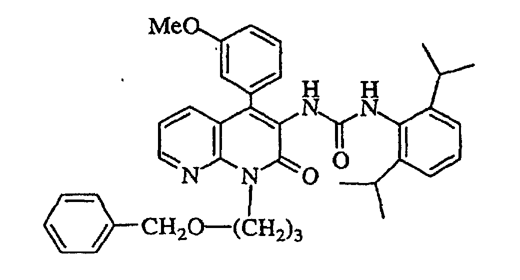

The title compound was obtained in the same manner as in Example 5 from 1-(3-benzyloxypropyl)-4-(3-methoxyphenyl)-1,2-dihydro-2-oxo-1,8-naphthyridine-3-carboxylic acid and 2,6-diisopropylaniline.
¹H-NMR δ (CD₃OD) 8.63 (1H, dd, J=4.6Hz, 2.0Hz), 7.75 (1H, dd, J=7.9Hz, 2.0Hz), 7.48 (1H, dd, J=8.3Hz, 7.6Hz), 7.03-7.40 (10H, m), 6.90-7.00 (2H, m), 4.82 (2H, t, J=7.3Hz), 4.52 (2H, s), 3.87 (3H, s), 3.71 (2H, t, J=5.9Hz), 3.03 (2H, sept, J=6.6Hz), 2.18 (2H, m), 1.14 (12H, brd, J=6.6Hz)

### Example 62

### Preparation of N-[1-(3-hydroxypropyl)-4-(3-methoxyphenyl)-1,2-dihydro-2-oxo-1,8-naphthyridin-3-yl]-N'-(2,6-diisopropylphenyl)urea

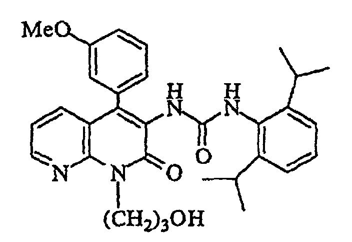

To a solution of N-[1-(3-benzyloxypropyl)-4-(3-methoxyphenyl)-1,2-dihydro-2-oxo-1,8-naphthyridin-3-yl]-N'-(2,6-diisopropylphenyl)urea (1.31 g, 2.12 mmol) in ethanol (80 ml) was added 10 % palladium-carbon (150 mg), and the mixture was stirred at room temperature under hydrogen atmosphere for three hours. To the mixture was added 12N hydrochloric acid (1 ml), and the mixture was further stirred at room temperature under hydrogen atmosphere for two hours. The mixture was filtered through a cerite pad, and the filtrate was concentrated under reduced pressure. The residue was purified by silica gel column chromatography (1-3 % methanol in chloroform) to give the title compound (1.12 g, 2.12 mmol).
¹H-NMR δ (CD₃OD) 8.65 (1H, dd, J=4.6Hz, 1.7Hz), 7.79 (1H, dd, J=7.9Hz, 1.7Hz), 7.49 (1H, dd, J=8.2Hz, 8.2Hz), 7.05-7.35 (5H, m), 6.95-7.04 (2H, m), 4.79 (2H, t, J=7.3Hz), 3.87 (3H, s), 3.71 (2H, t, J=6.3Hz), 3.03 (2H, sept, J=6.3Hz), 2.10 (2H, m), 1.15 (12H, brd, J=6.3Hz)

### Example 63

### Preparation of N-[(1-tert-butoxycarbonylmethyl)-4-(3-methoxyphenyl)-1,2-dihydro-2-oxo-1,8-naphthyridin-3-yl]-N'-(2,6-diisopropylphenyl)urea

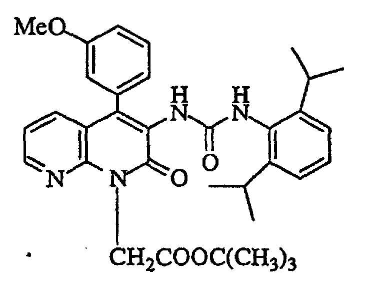

The title compound was obtained in the same manner as in Example 58 from N-[4-(3-methoxyphenyl)-1,2-dihydro-2-oxo-1,8-naphthyridin-3-yl]-N'-(2,6-diisopropylphenyl)urea and tert-butyl bromoacetate.
¹H-NMR δ (CD₃OD) 8.59 (1H, dd, J=4.6Hz, 1.7Hz), 7.78 (1H, dd, J=7.9Hz, 1.9Hz), 7.51 (1H, dd, J=7.9Hz, 7.9Hz), 6.95-7.35 (7H, m), 5.32 (2H, s), 3.88 (3H, s), 2.93-3.12 (2H, m), 1.53 (9H, s), 1.14 (12H, d, J=6.6Hz)

### Example 64

### Preparation of N-[1-carboxymethyl-4-(3-methoxyphenyl)-1,2-dihydro-2-oxo-1,8-naphthyridin-3-yl]-N'-(2,6-diisopropylphenyl)urea

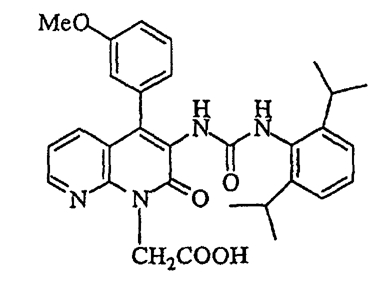

To a solution of N-[(1-tert-butoxycarbonylmethyl)-4-(3-methoxyphenyl)-1,2-dihydro-2-oxo-1,8-naphthyridin-3-yl]-N'-(2,6-diisopropylphenyl)urea (110 mg, 0.19 mmol) in methylene chloride (20 ml) was added trifluoroacetic acid (1 ml, 13 mmol), and the mixture was stirred at room temperature for 1.5 hour. The mixture was concentrated under reduced pressure, diluted with water, and basified with 4N aqueous NaOH solution, and washed with ethyl acetate. The aqueous layer was acidified with 2N aqueous HCl solution, extracted with ethyl acetate, washed with a saturated aqueous sodium chloride solution, and dried over anhydrous magnesium sulfate. The residue was concentrated under reduced pressure, and purified by preparative thin layer chromatography to give the title compound (41 mg, 0.77 mmol).
¹H-NMR δ (CD₃OD) 8.50-8.65 (1H, m), 7.78 (1H, d, 1=7.6Hz), 7.50 (1H, dd, J=7.9Hz, 7.9Hz), 6.96-7.38 (7H, m), 5.40 (2H, s), 3.88 (3H, s), 2.92 (2H, m), 1.14 (12H, d, J=6.3Hz)

### Example 65

### Preparation of N-[1-(2-hydroxyethyl)-4-(3-methoxyphenyl)-1,2-dihydro-2-oxo-1,8-naphthyridin-3-yl]-N'-(2,6-diisopropylphenyl)urea

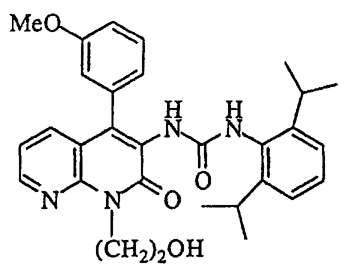

The title compound was obtained in the same manner as in Example 58 from N-[4-(3-methoxyphenyl)-1,2-dihydro-2-oxo-1,8-naphthyridin-3-yl]-N'-(2,6-diisopropylphenyl)urea and 2-bromoethanol.
¹H-NMR δ (CD₃OD) 8.63 (1H, dd, J=4.6Hz, 1.6Hz), 7.76 (1H, dd, J=7.9Hz, 1.7Hz), 7.50 (1H, dd, J=7.9Hz, 7.9Hz), 6.95-7.35 (7H, m), 4.89 (2H, t, J=6.3Hz), 3.98 (2H, t, J=6.3Hz), 3.87 (3H, s), 2.92-3.13 (2H, m), 1.15 (12H, d, J=6.6Hz)

### Example 66

### Preparation of N-[1-(4-pyridylmethyl)-4-(3-methoxyphenyl)-1,2-dihydro-2-oxo-1,8-naphthyridin-3-yl]-N'-(2,6-diisopropylphenyl)urea

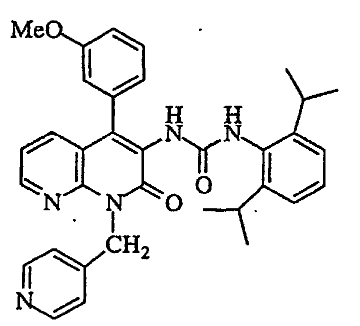

The title compound was obtained in the same manner as in Example 58 from N-[4-(3-methoxyphenyl)-1,2-dihydro-2-oxo-1,8-naphthyridin-3-yl]-N'-(2,6-diisopropylphenyl)urea and 4-picolyl chloride hydrochloride.
¹H-NMR δ (CD₃OD) 8.57 (1H, dd, J=4.6Hz, 1.7Hz), 8.40-8.50 (2H, m), 7.97 (1H, dd, J=7.9Hz, 1.7Hz), 7.51 (1H, dd, J=8.6Hz, 7.9Hz), 7.38-7.48 (2H, m), 6.99-7.35 (7H, m), 5.94 (2H, s), 3.88 (3H, s), 2.93-3.13 (2H, m), 1.14 (12H, d, J=6.3Hz)

### Example 67

### Preparation of N-[1-(3-dimethylaminopropyl)-4-(3-methoxyphenyl)-1,2-dihydro-2-oxo-1,8-naphthyridin-3-yl]-N'-(2,6-diisopropylphenyl)urea

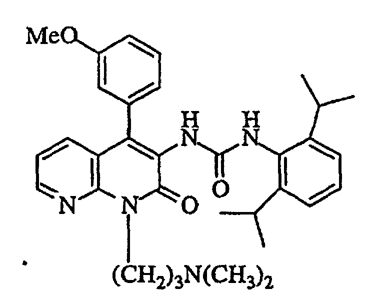

To a solution of N-[1-(3-aminopropyl)-4-(3-methoxyphenyl)-1,2-dihydro-2-oxo-1,8-naphthyridin-3-yl]-N'-(2,6-diisopropylphenyl)urea (100 mg, 0.19 mmol) in methanol (5 ml) were added successively 30 % HCl in ethanol (1 ml), 37 % aqueous formaldehyde solution (46 mg, 0.57 mmol) and sodium borohydride (36 mg, 0.57 mmol), and the mixture was stirred at room temperature for three hours. The mixture was poured into water, and the mixture was basified with conc. aqueous ammonia, and extracted with ethyl acetate. The extract was washed with a saturated aqueous sodium chloride solution, and dried over anhydrous magnesium sulfate. The residue was concentrated under reduced pressure, and purified by preparative thin layer chromatography to give the title compound (79 mg, 0.14 mmol).
¹H-NMR δ (CD₃OD) 8.65 (1H, dd, J=4.6Hz, 1.7Hz), 7.77 (1H, dd, J=7.9H, 1.7Hz), 7.49 (1H, dd, J=7.9Hz, 7.9Hz), 6.95-7.35 (7H, m), 4.65-4.78 (2H, m), 3.87 (3H, s), 2.95-3.10 (2H, m), 2.52-2.68 (2H, m), 2.37 (6H, s), 2.00-2.15 (2H, m), 1.15 (12H, d, J=6.6Hz)

### Example 68

### Preparation of N-[1-(3-aminocarbonylpropyl)4-(3-methoxyphenyl)-1,2-dihydro-2-oxo-1,8-naphthyridin-3-yl]-N'-(2,6-diisopropylphenyl)urea

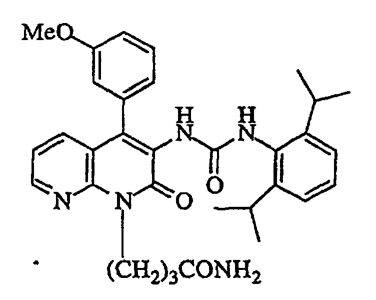

To a solution of N-[1-(3-cyanopropyl)-4-(3-methoxyphenyl)-1,2-dihydro-2-oxo-1,8-naphthyridin-3-yl]-N'-(2,6-diisopropylphenyl)urea (100 mg, 0.19 mmol) in methanol-acetone (3 ml) were added 10 % aqueous sodium carbonate solution (1 ml) and 30 % hydrogen peroxide (1 ml), and the mixture was stirred at room temperature for three hours. The mixture was poured into water, and extracted with ethyl acetate. The extract was washed with a saturated aqueous sodium chloride solution, and dried over anhydrous magnesium sulfate. The residue was concentrated under reduced pressure, and purified by preparative thin layer chromatography to give the title compound (96 mg, 0.17 mmol).
¹H-NMR δ (DMSO-d₆) 8.52-8.68 (1H, m), 7.68-7.85 (2H, m), 7.55-7.68 (1H, m), 6.60-7.50 (10H, m), 4.40-4.65 (2H, m), 3.77 (3H, s), 2.80-3.00 (2H, m), 2.10-2.30 (2H, m), 1.80-2.07 (2H, m), 1.03 (brs, 12H)

### Example 69

### Preparation of N-[1-(3-cyanopropyl)-4-{3-(2-pyridylmethoxy)phenyl}-1,2-dihydro-2-oxo-1,8-naphthyridin-3-yl]-N'-(2,6-diisopropylphenyl)urea

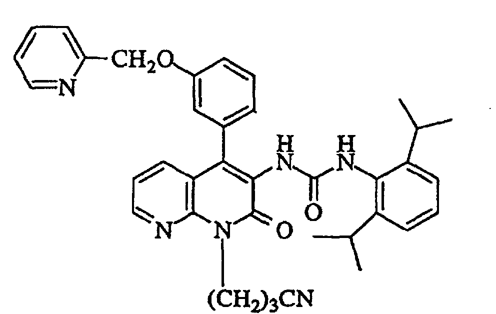

The title compound was obtained in the same manner as in Example 41 from N-[1-(3-cyanopropyl)-4-(3-hydroxyphenyl)-1,2-dihydro-2-oxo-1,8-naphthyridin-3-yl]-N'-(2,6-diisopropylphenyl)urea and 2-picolyl chloride hydrochloride.

Hydrochloride: m.p. 151-154°C

### Example 70

### Preparation of N-[1-(2-pyridylmethyl)-4-(3-methoxyphenyl)-1,2-dihydro-2-oxo-1,8-naphthyridin-3-yl]-N'-(2,6-diisopropylphenyl)urea

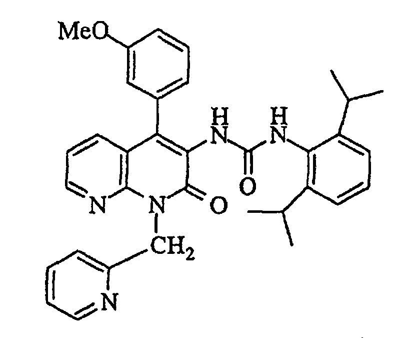

The title compound was obtained in the same manner as in Example 58 from N-[4-(3-methoxyphenyl)-1,2-dihydro-2-oxo-1,8-naphthyridin-3-yl]-N'-(2,6-diisopropylphenyl)urea and 4-picolyl chloride hydrochloride.
¹H-NMR δ (CD₃OD) 8.50 (1H, dd, J=4.6Hz, 1.7Hz), 8.44 (1H, m), 7.72-7.83 (2H, m), 7.52 (1H, dd, J=8.3Hz, 7.9Hz), 7.00-7.38 (9H, m), 6.02 (2H, s), 3.89 (3H, s), 2.90-3.13 (2H, m), 1.12 (12H, brs)

### Example 71

### Preparation of N-[1-(3-piperidinopropyl)-4-(3-methoxyphenyl)-1,2-dihydro-2-oxo-1,8-naphthyridin-3-yl]-N'-(2,6-diisopropylphenyl)urea

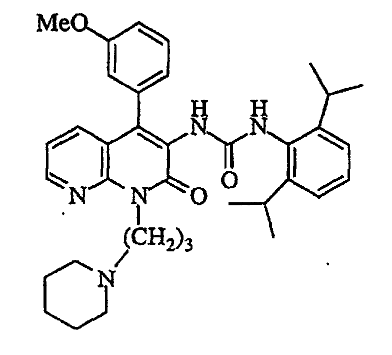

To a solution of N-(3-chloropropyl)piperidine hydrochloride (422 mg, 2.13 mmol) in DMF (30 ml) was added sodium bromide (330 mg, 3.20 mmol), and the mixture was stirred at about 120°C for one hour, and then cooled to about 20°C. To the mixture were added successively N-[4-(3-methoxyphenyl)-1,2-dihydro-2-oxo-1,8-naphthyridin-3-yl]-N'-(2,6-diisopropylphenyl)urea (100 mg, 0.21 mmol), potassium carbonate (147 mg, 1.06 mmol) and potassium iodide (35 mg, 0.21 mmol), and the mixture was stirred at about 50°C for three hours. To the mixture was added sodium bromide (660 mg, 6.40 mmol), and the mixture was stirred at about 80°C for 4.5 hours, and cooled. The mixture was poured into water, and the mixture was extracted with ethyl acetate, washed with a saturated aqueous sodium chloride solution, and dried over anhydrous magnesium sulfate. The residue was concentrated under reduced pressure, and purified by preparative thin layer chromatography to give the title compound (77 mg, 0.12 mmol).
¹H-NMR δ (CD₃OD) 8.63 (1H,dd, J=4.6Hz, 1.7Hz), 7.76 (1H, dd, J=7.9Hz, 1.7Hz), 7.49 (1H, dd, J=8.3Hz, 7.9Hz), 6.95-7.30 (7H, m), 4.71 (2H, t, J=7.3Hz), 3.87 (3H, s), 2.95-3.12 (2H, m), 2.40-2.80 (6H, m), 2.00-2.20 (2H, m), 1.40-1.80 (6H, m), 0.95-1.30 (12H, t, J=5.9Hz)

### Example 72

### Preparation of N-[1-(3-diethylaminopropyl)-4-(3-methoxyphenyl)-1,2-dihydro-2-oxo-1,8-naphthyridin-3-yl]-N'-(2,6-diisopropylphenyl)urea

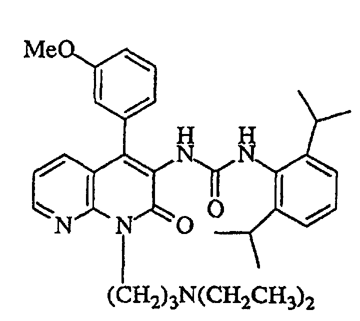

The title compound was obtained in the same manner as in Example 71 from N-[4-(3-methoxyphenyl)-1,2-dihydro-2-oxo-1,8-naphthyridin-3-yl]-N'-(2,6-diisopropylphenyl)urea and 3-diethylaminopropyl chloride hydrochloride.
¹H-NMR δ (CD₃OD) 8.64 (1H, dd, J=4.6Hz, 1.7Hz), 7.76 (1H, dd, J=7.9Hz, 1.7Hz), 7.50 (1H, dd, J=8.3Hz, 8.3Hz), 6.93-7.33 (7H, m), 4.71 (2H, t, J=7.3Hz), 3.88 (3H, s), 2.95-3.10 (2H, m), 2.85-2.93 (2H, m), 2.80 (4H, q, J=7.3Hz), 2.00-2.20 (2H, m), 1.00-1.30 (18H, m)

### Example 73

### Preparation of N-[1-butyl-4-{3-(3-diethylaminopropoxy)phenyl}-1,2-dihydro-2-oxo-1,8-naphthyridin-3-yl]-N'-(2,6-diisopropylphenyl)urea

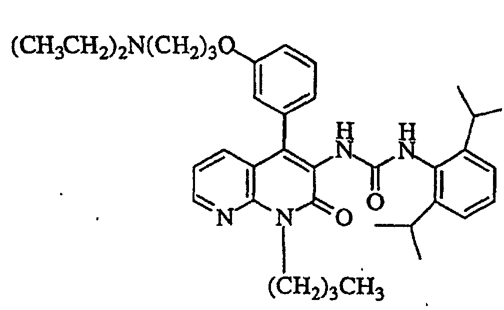

The title compound was obtained in the same manner as in Example 41 from N-[1-butyl-4-(3-hydroxyphenyl)-1,2-dihydro-2-oxo-1,8-naphthyridin-3-yl]-N'-(2,6-diisopropylphenyl)urea and 3-diethylaminopropyl chloride hydrochloride.
Hydrochloride:
¹H-NMR δ (DMSO-d₆) 9.90 (1H, brs), 8.50-8.70 (1H, m), 7.80 (1H, brs), 7.50-7.65 (1H, m), 7.35-7.45 (1H, m), 6.72-7.30 (7H, m), 4.40-4.62 (2H, m), 3.92-4.18 (2H, m), 2.70-3.22 (8H, m), 2.00-2.21 (2H, m), 1.56-1.83 (2H, m), 1.30-1.50 (2H, m), 0.60-1.30 (21H, m)

### Example 74

### Preparation of N-[1-butyl-4-[3-{2-(1-pyrrolidinyl)ethoxy}phenyl]-1,2-dihydro-2-oxo-1,8-naphthyridin-3-yl]-N'-(2,6-diisopropylphenyl)urea

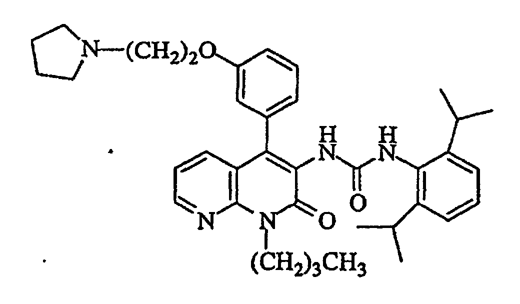

The title compound was obtained in the same manner as in Example 41 from N-[1-butyl-4-(3-hydroxyphenyl)-1,2-dihydro-2-oxo-1,8-naphthyridin-3-yl]-N'-(2,6-diisopropylphenyl)urea and 2-(1-pyrrolidinyl)ethyl chloride hydrochloride.
Hydrochloride:
¹H-NMR δ (DMSO-d₆) 8.55-8.65 (1H, m), 7.75-7.90 (1H, m), 7.55-7.65 (1H, m), 7.35-7.48 (1H, m), 6.85-7.20 (8H, m), 4.43-4.60 (2H, m), 4.25-4.30 (2H, m), 3.40-3.65 (4H, m), 2.75-3.15 (4H, m), 1.56-2.10 (6H, m), 1.25-1.53 (2H, m), 0.80-1.20 (15H, m)

### Example 75

### Preparation of N-[1-butyl-4-{3-(2-piperidinoethoxy)phenyl}-1,2-dihydro-2-oxo-1,8-naphthyridin-3-yl]-N'-(2,6-diisopropylphenyl)urea

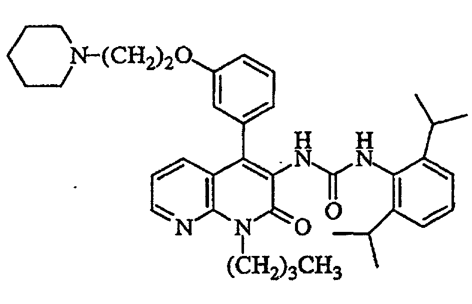

The title compound was obtained in the same manner as in Example 41 from N-[1-butyl-4-(3-hydroxyphenyl)-1,2-dihydro-2-oxo-1,8-naphthyridin-3-yl]-N'-(2,6-diisopropylphenyl)urea and 2-piperidinoethyl chloride hydrochloride.
Hydrochloride: m.p. 154-156°C

### Example 76

### Preparation of N-[1-(3-tert-butoxycarbonylaminopropyl)-4-(3-methoxyphenyl)-1,2-dihydro-2-oxo-1,8-naphthyridin-3-yl]-N'-(2,6-diisopropylphenyl)-urea

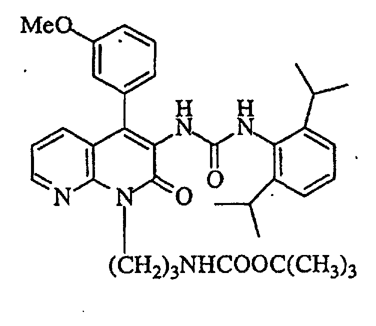

The title compound was obtained in the same manner as in Example 58 from N-[4-(3-methoxyphenyl)-1,2-dihydro-2-oxo-1,8-naphthyridin-3-yl]-N'-(2,6-diisopropylphenyl)urea and 3-tert-butoxycarbonylaminopropyl iodide.
¹H-NMR δ (CD₃OD) 8.64 (1H,dd, J=5.0Hz, 1.7Hz), 7.77 (1H, dd, J=8.3Hz, 1.7Hz), 7.49 (1H, dd, J=8.3Hz, 7.6Hz), 6.95-7.35 (7H, m), 4.74 (2H, t, J=6.9Hz), 3.87 (3H, s), 3.20 (2H, t, J=6.6Hz), 2.95-3.10 (2H, m), 1.90-2.10 (2H, m), 1.49 (9H, s), 1.15 (12H, brd, J=6.3Hz)

### Example 77

### Preparation of N-[1-{3-(imidazol-1-yl)propyl}-4-(3-methoxyphenyl)-1,2-dihydro-2-oxo-1,8-naphthyridin-3-yl]-N'-(2,6-diisopropylphenyl)urea

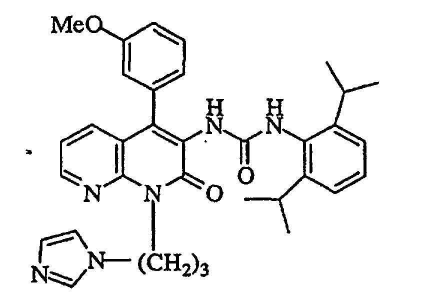

The title compound was obtained in the same manner as in Example 58 from N-[4-(3-methoxyphenyl)-1,2-dihydro-2-oxo-1,8-naphthyridin-3-yl]-N'-(2,6-diisopropylphenyl)urea and 3-(imidazol-1-yl)propyl bromide hydrobromide.
Hydrochloride:
¹H-NMR δ (DMSO-d₆) 9.20 (1H, brs), 8.57 (1H, dd, J=4.6Hz, 1.3Hz), 7.55-8.00 (6H, m), 7.37-7.48 (1H, m), 7.22-7.32 (1H, m), 7.11-7.18 (1H, m), 6.98-7.09 (3H, m), 6.86-6.94 (2H, m), 4.47-4.62 (2H, m), 4.20-4.40 (2H, m), 3.38 (3H, s), 2.84-2.98 (2H, m), 2.25-2.40 (2H, m), 1.02 (12H, d, J=5.9Hz)

### Example 78

### Preparation of N-[1-butyl-4-{3-(2-morpholinoethoxy)phenyl}-1,2-dihydro-2-oxo-1,8-naphthyridin-3-yl]-N'-(2,6-diisopropylphenyl)urea

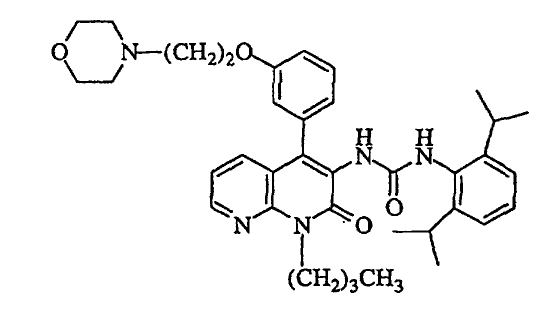

The title compound was obtained in the same manner as in Example 41 from N-[1-butyl-4-(3-hydroxyphenyl)-1,2-dihydro-2-oxo-1,8-naphthyridin-3-yl]-N'-(2,6-diisopropylphenyl)urea and 2-morpholinoethyl chloride hydrochloride.
¹H-NMR δ (CD₃OD) 8.64 (1H, d, J=4.6Hz, 1.7Hz), 7.75 (1H, d, J=7.9Hz, 1.7Hz), 7.48 (1H, dd, J=8.2Hz, 7.9Hz), 6.95-7.20 (7H, m), 4.60-4.70 (2H, m), 4.21 (2H, t, J=5.6Hz), 3.65-3.80 (4H, m), 2.94-3.10 (2H, m), 2.85 (2H, t, J=5.6Hz), 2.52-2.70 (4H, m), 1.70-1.93 (2H, m), 1.43-1.65 (2H, m),1.14 (12H, d, J=6.6Hz), 1.06 (3H, t, J=7.3Hz)

### Reference Example 1

Preparation of 3-amino-4-(2-chlorophenyl)-1,7-naphthyridine

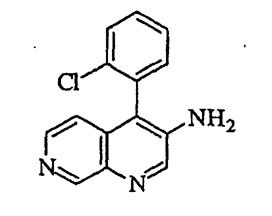

### (a) Preparation of 2-hydroxy-N-[α-(3-amino-4-pyridyl)-2-chlorobenzylidine]-ethylamine

A mixture of 3-amino-4-(2-chlorobenzoyl)pyridine (5.65 g, 24 mmol), 2-methylimidazole hydrochloride (4.7 g, 40 mmol) and ethanolamine (12.2 g, 200 mmol) was melted with heating at about 130°C for 5 hours. After allowed to stand for cooling, to the mixture was added water. The precipitated crystals were collected by filtration; and recrystallized from ethyl acetate to give the title compound (6.28 g, 22 mmol) as pale yellow crystals.
m.p. 175-178°C
¹H-NMR δ (DMSO-d₆) 8.18 (1H, s), 7.47-7.65 (6H, m), 7.24-7.27 (1H, m), 6.32 (1H, d, J=5.3Hz), 4.73 (1H, t, J=5.6Hz, disappeared with D₂O-exchange), 3.62-3.68 (2H, m), 3.17-3.30 (2H, m)
IR (KBr) 3387, 1614, 1434,1308,1239, 1055 cm⁻¹

### (b) Preparation of 2,2-diethoxy-N-[α-(3-amino-4-pyridyl)-2-chlorobenzylidene]ethylamine

A solution of 2-hydroxy-N-[α-(3-amino-4-pyridyl)-2-chlorobenzylidene]-ethylamine (6.28 g, 22 mmol), aminoacetaldehyde diethyl acetal (10 g, 75.1 mmol) and acetic acid (3 ml) in ethanol (150 ml) was refluxed for 30 hours. The mixture was concentrated under reduced pressure, and to the residue was added water, and the mixture was extracted with ethyl acetate. The extract was washed with water, washed with a saturated aqueous sodium chloride solution, dried over anhydrous sodium sulfate, and concentrated under reduced pressure. The residue was purified by column chromatography (silica gel; methanol:chloroform = 1:9) to give the title compound (4.27 g, 11.9 mmol) as a pale yellow solid.
m.p. 94-95°C
¹H-NMR δ (CDCl₃) 8.18 (1H, s), 7.74 (1H, d, J=5.28Hz), 7.35-7.51 (3H, m), 7.07-7.13 (1H, m), 6.64 (2H, brs), 6.50 (1H, d, J=5.0Hz), 4.88 (1H, dd, J=5.9Hz, 5.3Hz), 3.33-3.76 (6H, m), 1.21 (3H, t, J=7.0Hz), 1.20 (3H, t, J=7.3Hz)
IR (KBr) 3393, 2978, 1608, 1594, 1429, 1236 cm⁻¹

### (c) Preparation of 3-amino-4-(2-chlorophenyl)-1,7-naphthyridine

2,2-Diethoxy-N-[α-(3-amino-4-pyridyl)-2-chlorobenzylidene]ethylamine (4.00 g, 11.17 mmol) was dissolved in 10 % hydrogen chloride in ethanol (60 ml), and the mixture was refluxed for 5 hours. The mixture was concentrated under reduced pressure, and to the residue was added a saturated aqueous sodium hydrogen carbonate solution, and the mixture was extracted with ethyl acetate. The extract was washed with water, washed with a saturated aqueous sodium chloride solution, dried over anhydrous sodium sulfate, and concentrated under reduced pressure. The residue was purified by column chromatography (silica gel; methanol:chloroform =1:19) to give the title compound (0.99 g, 3.87 mmol) as a colorless solid.
¹H-NMR δ (CDCl₃) 8.30 (1H, s), 7.86 (1H, dd, J=5.3Hz, 0.7Hz), 7.27-7.50 (5H, m), 6.95 (1H, d, J=5.3Hz), 6.28 (2H, br)

### Reference Example 2

### Preparation of 4-(2-chlorophenyl)-1,2-dihydro-1-methyl-2-oxo-1,8-naphthyridine-3-carboxylic acid

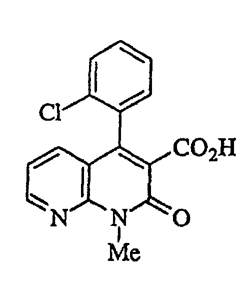

### (a) Preparation of ethyl 4-(2-chlorophenyl)-1,2-dihydro-2-oxo-1,8-naphthyridine-3-carboxylate

A mixture of 2-amino-3-(2-chlorlbenzoyl)pyridine (3.91 g, 16.8 mmol), diethyl malonate (4.04 g, 25.2 mmol) and pyridine (0.33 g, 4.2 mmol) was heated with stirring at about 170°C for five hours. After allowed to stand for cooling, the precipitated crystals were recrystallized from ethanol to give the title compound (4.73 g, 14.4 mmol) as colorless crystals.
m.p. 218-221°C
¹H-NMR δ (CDCl₃) 11.53 (1H, brs), 8.76 (1H, dd, J=5.0Hz, 1.32Hz), 7.26-7.57 (5H, m), 7.17 (1H, dd, J=7.9Hz, 5.0Hz), 4.04-4.17 (2H, m), 0.97 (3H, t, J=7.0Hz)
IR (KBr) 1739, 1667, 1613, 1568, 1466, 1425, 1375 cm⁻¹

### (b) Preparation of ethyl 1-methyl-4-(2-chlorophenyl)-1,2-dihydro-2-oxo-1,8-naphthyridine-3-carboxylate

To a solution of ethyl 4-(2-chlorophenyl)-1,2-dihydro-2-oxo-1,8-naphthyridine-3-carboxylate (4.50, 13.7 mmol) in DMF (50 ml) was added sodium hydride (60 % oily, 547 mg, 13.7 mg) at room temperature, and the mixture was stirred for 0.5 hour. To the mixture was added methyl iodide (1.9 g, 13.7 mmol) at 0°C to 5°C, and the mixture was stirred at the same temperature for 0.5 hour, and then stirred at room temperature for five hours. The mixture was poured into water, and extracted with ethyl acetate. The extract was washed with water, washed with a saturated aqueous sodium chloride solution, dried over anhydrous sodium sulfate, and concentrated under reduced pressure to give the title compound (4.60 g, 13.4 mmol), which was used without further isolation in the subsequent reaction.
¹H-NMR δ (CDCl₃) 8.65 (1H, dd, J=4.6Hz, 1.7Hz), 7.29-7.56 (5H, m), 7.10-7.15 (1H, m), 4.07-4.13 (2H, m), 3.92 (3H, s), 0.98 (3H, t, J=7.0Hz)

### (c) Preparation of 1-methyl-4-(2-chlorophenyl)-1,2-dihydro-2-oxo-1,8-naphthyridine-3-carboxylic acid

To a solution of ethyl 1-methyl-4-(2-chlorophenyl)-1,2-dihydro-2-oxo-1,8-naphthyridine-3-carboxylate (4.6 g, 13.4 mmol) in ethanol (20 ml) was added sodium hydroxide (2.1 g, 52.5 mmol), and the mixture was refluxed for 0.5 hour. The mixture was diluted with water, and the pH value thereof was adjusted to pH 4 with 2N aqueous hydrochloric acid. The mixture was extracted with ethyl acetate, and the extract was washed with a saturated aqueous sodium chloride solution, dried over anhydrous sodium sulfate, and concentrated under reduced pressure. The precipitated crystals were recrystallized from ethyl acetate to give the title compound (3.52 g, 11.2 mmol) as colorless crystals.
m.p. 178-180°C
¹H-NMR δ (CDCl₃) 8.80 (1H, dd, J=4.6Hz, 2.0Hz), 7.39-7.57 (4H, m), 7.24-7.29 (1H, dd, J=7.9Hz, 4.6Hz), 7.11 (1H, dd, J=7.9Hz, 2.0Hz), 4.07 (3H, s)
IR (KBr) 1747, 1612, 1576, 1472, 1446, 1342 cm⁻¹

### Reference Example 3

### Preparation of 1-methyl-4-(2-chlorophenyl)-1,2-dihydro-2-oxo-1,7-naphthyridine-3-carboxylic acid

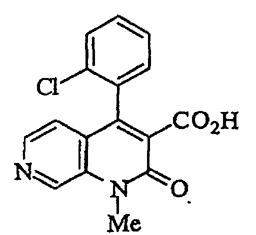

The title compound was obtained in the same manner as in Reference Example 2.
m.p. >250°C
¹H-NMR δ (DMSO-d₆) 9.08 (1H, s), 8.41 (1H, d, J=5.3Hz), 7.50-7.69 (3H, m), 7.40 (1H, dd, J=7.3Hz, 1.7Hz), 6.86 (1H, d, J=5.3Hz), 3.82 (3H, s)
IR (KBr) 1722, 1657, 1434, 1295, 1251 cm⁻¹

### Reference Example 4

### Preparation of 1-methyl-4-(3-methoxyphenyl)-1,2-dihydro-2-oxo-1,8-naphthyridine-3-carboxylic acid

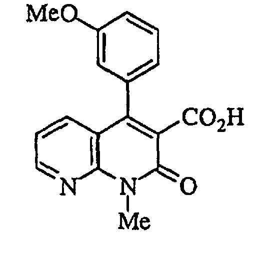

The title compound was obtained in the same manner as in Reference Example 2.
m.p. 196-197°C
¹H-NMR δ (CDCl₃) 8.77 (1H, dd, J=4.6Hz, 2.0Hz), 7.66 (1H, dd, J=8.3Hz, 2.0Hz), 7.44 (1H, t, J=8.3Hz), 7.22-7.25 (1H, m), 7.01-7.05 (1H, m), 6.70-6.78 (2H, m), 4.04 (3H, s), 3.83 (3H, s)
IR (KBr) 1734,1624,1604, 1573, 1462, 1249 cm⁻¹

### Reference Example 5

Preparation of 4-(3-methoxyphenyl)-1,2-dihydro-2-oxo-1,8-naphthyridine-3-carboxylic acid

The title compound was obtained in the same manner as in Reference Example 2.
¹H-NMR δ (CDCl₃) 8.84 (1H, d, J=3.0Hz), 7.69 (1H, d, J=8.2Hz), 7.46 (1H, dd, J=7.9Hz, 7.9Hz), 7.28-7.33 (1H, m), 7.05 (1H, dd, J=8.3Hz, 1.7Hz), 6.73-6.80 (2H, m), 3.84 (3H, s).

### Reference Example 6

Preparation of 1-butyl-4-(3-methoxyphenyl)-1,2-dihydro-2-oxo-1,8-naphthyridine-3-carboxylic acid

The title compound was obtained in the same manner as in Reference Example 2.
¹H-NMR δ (CDCl₃) 8.76 (1H, dd, J=4.6Hz, 2.0Hz), 7.65 (1H, dd, J=7.9Hz, 2.0Hz), 7.43 (1H, dd, J=7.9Hz, 7.9Hz), 7.22 (1H, dd, J=7.9Hz, 4.6Hz), 7.02 (1H, dd, J=7.6Hz, 1.6Hz), 6.70-6.78 (2H, m), 4.68-4.74 (2H, m), 3.82 (3H, s), 1.77-1.88 (2H, m), 1.45-1.59 (2H, m), 1.03 (3H, t, J=7.3Hz)

### Reference Example 7

### Preparation of 1-(2-methoxyethyl)-4-(3-methoxyphenyl)-1,2-dihydro-2-oxo-1,8-naphthyridine-3-carboxylic acid

The title compound was obtained in the same manner as in Reference Example 2.
¹H-NMR δ (CDCl₃) 8.76 (1H, dd, J=4.6Hz, 2.0Hz), 7.65 (1H, dd, J=7.9Hz, 2.0Hz), 7.43 (1H, dd, J=7.9Hz, 7.9Hz), 7.23 (1H, dd, J=8.3Hz, 4.6Hz), 7.03 (1H, dd, J=7.9Hz, 2.6Hz), 6.71-6.79 (2H, m), 4.99 (2H, t, J=6.0Hz), 3.88 (2H, t, J=6.0Hz), 3.83 (3H, s), 3.42 (3H, s)

### Reference Example 8

### Preparation of 1-isopropyl-4-(3-methoxyphenyl)-1,2-dihydro-2-oxo-1,8-naphthyridine-3-carboxylic acid

The title compound was obtained in the same manner as in Reference Example 2.
¹H-NMR 8 (CDCl₃) 8.73 (1H, dd, J=4.6Hz, 1.0Hz), 7.64 (1H, dd, J=7.9Hz, 2.0Hz), 7.43 (1H, dd, J=7.9Hz, 7.9Hz), 7.21 (1H, dd, J=7.9Hz), 6.99-7.04 (1H, m), 6.70-6.78 (2H, m), 6.27 (1H, br), 3.82 (3H, s), 1.73 (6H, d, J=6.9Hz)

### Reference Example 9

### Preparation of 1-methyl-4-(4-methoxyphenyl)-1,2-dihydro-2-oxo-1,8-naphthyridine-3-carboxylic acid

The title compound was obtained in the same manner as in Reference Example 2.
¹H-NMR 8 (CDCl₃) 8.76 (1H, dd, J=4.6Hz, 1.7Hz), 7.25 (1H, dd, J=7.9Hz, 4.6Hz), 7.11 (2H, d, J=8Hz), 7.05 (2H, d, J=8.3Hz), 4.04 (3H, s), 3.89 (3H, s)

### Reference Example 10

### Preparation of 1-(3-cyanopropyl)-4-(4-methoxyphenyl)-1,2-dihydro-2-oxo-1,8-naphthyridine-3-carboxylic acid

The title compound was obtained in the same manner as in Reference Example 2.
¹H-NMR δ (CDCl₃) 8.76 (1H, dd, J=4.6Hz, 2.0Hz), 7.69 (1H, dd, J=7.9Hz, 2.0Hz), 7.44 (1H, dd, J=7.9Hz, 7.9Hz), 7.25-7.29 (1H, m), 7.03 (1H, d, J=7.9Hz), 6.76 (1H, d, J=7.9Hz), 6.71 (1H, s), 4.86 (2H, t, J=6.9Hz), 3.83 (3H, s), 2.56 (2H, t, J=6.9Hz), 2.29 (2H, m)

### Reference Example 11

### Preparation of 1-methyl-4-(2-methoxyphenyl)-1,2-dihydro-2-oxo-1,8-naphthyridine-3-carboxylic acid

The title compound was obtained in the same manner as in Reference Example 2.
¹H-NMR δ (CDCl₃) 8.75 (1H, dd, J=4.6Hz, 2.0Hz), 7.63 (1H, dd, J=8.3Hz, 2.0Hz), 7.44-7.51 (1H, m), 7.23 (1H, dd, J=7.9Hz, 4.6Hz), 6.98-7.13 (3H, m), 4.04 (3H, s), 1.70 (3H, s)

### Reference Example 12

### Preparation of 1-pentyl-4-(3-methoxyphenyl)-1,2-dihydro-2-oxo-1,8-naphthyridine-3-carboxylic acid

The title compound was obtained in the same manner as in Reference Example 2.
¹H-NMR δ (CDCl₃) 8.76 (1H, dd, J=4.6Hz, 1.7Hz), 7.64 (1H, dd, J=8.3Hz, 1.7Hz), 7.42 (1H, dd, J=7.9Hz, 7.9Hz), 7.22 (1H, dd, J=8.3Hz, 4.6Hz), 7.02 (1H, dd, J=7.9Hz, 2.3Hz), 6.73 (1H, d, J=7.9Hz), 6.71 (1H, s), 4.70 (2H, t, J=7.6Hz), 1.84 (2H, br), 1.46 (4H, br), 0.95 (3H, t, J=6.9Hz)

### Reference Example 13

### Preparation of 1-(3-methylbutyl)-4-(3-methoxyphenyl)-1,2-dihydro-2-oxo-1,8-naphthyridine-3-carboxylic acid

The title compound was obtained in the same manner as in Reference Example 2.
¹H-NMR δ (CDCl₃) 8.76 (1H, dd, J=4.6Hz, 2.0Hz), 7.64 (1H, dd, J=8.3Hz, 2.0Hz), 7.42 (1H, dd, J=7.9Hz, 7.9Hz), 7.22 (1H, dd, J=8.3Hz, 4.6Hz), 7.02 (1H, dd, J=8.3Hz, 2.0Hz), 6.76 (1H, d, J=7.6Hz), 6.71 (1H, d, J=2.0Hz), 4.73 (2H, t, J=7.9Hz), 3.82 (3H, s), 1.67-1.84 (3H, m), 1.06 (6H, d, J=6.6Hz)

### Reference Example 14

### Preparation of 1-butyl-4-(2-pyridyl)-1,2-dihydro-2-oxo-1,8-naphthyridine-3-carboxylic acid

The title compound was obtained in the same manner as in Reference Example 2.
¹H-NMR δ (CDCl₃) 8.80 (1H, dd, J=4.6Hz, 2.0Hz), 8.76 (1H, dd, J=4.6Hz, 2.0Hz), 8.45 (1H, d, J=1.3Hz), 7.55-7.59 (2H, m), 7.45-7.50 (1H, m), 7.26 (1H, dd, J=7.9Hz, 4.6Hz), 4.73 (2H, t, J=7.6Hz), 1.78-1.89 (2H, m), 1.46-1.59 (2H, m), 1.07 (3H, t, J=7.3Hz)

### Reference Example 15

Preparation of 1-(3-benzyloxypropyl)-4-(3-methoxyphenyl)-1,2-dihydro-2-oxo-1,8-naphthyridine-3-carboxylic acid

The title compound was obtained in the same manner as in Reference Example 2.
¹H-NMR δ (CDCl₃) 8.74 (1H, dd, J=4.6Hz, 1.7Hz), 7.62 (1H, dd, J=7.9Hz, 1.7Hz), 7.43 (1H, dd, J=7.9Hz, 7.9Hz), 7.20-7.37 (5H, m), 7.21 (1H, dd, J=7.9Hz, 4.6Hz), 7.02 (1H, dd, J=7.9Hz, 2.6Hz), 6.60-6.75 (2H, m), 4.87 (2H, t, J=7.3Hz), 4.51 (2H, s), 3.82 (3H, s), 3.69 (2H, t, J=5.9Hz), 2.19 (2H, dd, J=7.3Hz, 5.9Hz)

### EFFECTS OF INVENTION

The naphthyridine derivative of the present invention or an acid addition salt thereof strongly inhibits ACAT activity in a specimen of rabbit liver or in rat peritoneal-derived macrophage. Therefore, the present compound or an acid addition salt thereof is useful not only as an agent for treatment of hyperlipidemia, but also in the prophylaxis or treatment of atherosclerosis per se or various diseases accompanied by atherosclerosis, for example, cerebral infarction, cerebral thrombosis, transient cerebral ischemia, angina pectoris, myocardial infarction, peripheral thrombus or occlusion.

The ACAT inhibitory activity of the present compounds were evaluated as follows.

### Experiment

### 1. Assay of ACAT inhibitory activity in a specimen prepared from rabbit liver

An enzyme specimen ACAT was prepared according to the method disclosed in the literature: J. Lipid. Research, **30**, 681-690, 1989, from the liver of New Zealand white rabbit which had been fed with 1 % cholesterol feed for one month. The ACAT activity was determined according to the method disclosed in the literature: J. Lipid Research. **24**, 1127-1134, 1983, i.e., using radioactive [1-¹⁴C]oleoyl-CoA and endogenous cholesterol contained in the liver microsome, and calculated from the radioactivity of the labeled cholesterol oleate. The results are shown in Table 1.

**Table 1**

| Test compound (Example No.) | ACAT inhibitory rate (%) 10⁻⁶ M |
|---|---|
| 4 | 87 |
| 6 | 82 |

### 2. Assay of ACAT inhibitory activity in the macrophage derived from rat peritoneal

The rat peritoneal-derived macrophage was prepared according to the method disclosed in the literature: Biochimica et Biophysica Acta, 1126, 73-80, 1992. The ACAT activity was determined by a modified method of the method disclosed in the above literature: Biochimica et Biophysica Acta, 1126, 73-80, 1992, i.e., using radioactive [9,10-H]oleic acid and exogenous cholesterol contained in the liposome which was re-constituted according to the method disclosed in the literature: Biochimica Biophysica Acta, 1213, 127-134, 1994, and calculated from the labeled cholesterolyl oleate. The results are shown in Table 2.

**Table 2**

| Test compound (Example No.) | ACAT inhibitory rate (%) 10⁻⁶ M |
|---|---|
| 4 | 88 |
| 7 | 94 |

## Claims

1. A naphthyridine derivative of the formula: 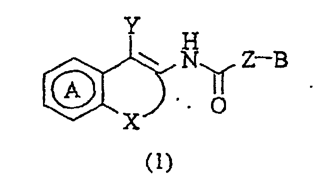 wherein Ring A is a substituted or unsubstituted pyridine ring selected from the following formulae (a), (b) and (c): 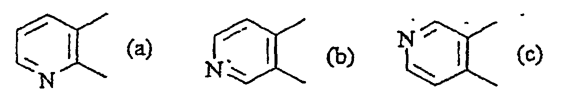 wherein the substituent of the pyridine ring is selected from the group consisting of a C₁-C₆ alkyl group, a halogen atom, a cyano group, a trifluoromethyl group, a nitro group, an amino group, a mono-C₁-C₆ alkylamino group, a di-C₁-C₆ alkylamino group, a hydroxy group, a C₁-C₆ alkoxy group, a C₁-C₆ alkylthio group, a C₁-C₆ alkylsulfinyl group, and a C₁-C₆ alkylsulfonyl group,
X is a group of the formula: 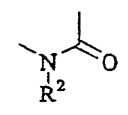 wherein R² is an alkyl group, a substituted alkyl group, an alkenyl group, or a substituted alkenyl group,
Z is a group: -NH-
B is an aromatic group or a substituted aromatic group, and Y is a phenyl group, a phenyl group substituted by a C₁-C₆ alkoxy group, or a phenyl group substituted by a group of the formula:
-D¹-E-F
(D¹ is a direct bond, an oxygen atom, a sulfur atom or a group of the formula: -NR³- (R³ is a hydrogen atom or a C₁-C₆ alkyl group), E is a divalent hydrocarbon group having 1 to 6 carbon atoms and optionally containing an unsaturated bond, or a phenylene group, F is a hydroxy group, a carboxyl group, a C₁-C₆ alkoxycarbonyl group, a benzyloxycarbonyl group, a halogen atom, a cyano group, a benzyloxy group, a C₁-C₆ alkoxy group, a C₁-C₆ alkanoyloxy group, a C₁-C₆ alkylthio group, a C₁-C₆ alkylsulfinyl group, a C₁-C₆ alkylsulfonyl group, a C₁-C₆ alkanoylamino group, a C₁-C₆ alkylsulfonamido group, a phthalimido group, a heteroaryl group, a group of the formula: -NR⁴R⁵ (R⁴ and R⁵ are independently a hydrogen atom or a C₁-C₆ alkyl group, or R⁴ and R⁵ may combine with the adjacent nitrogen atom to which they bond to form a saturated 5- to 7-membered cyclic amino group optionally having one -NR⁸- (R⁸ is a hydrogen atom, a C₁-C₆ alkyl group, a phenyl group, or a benzyl group) or one oxygen atom in the cycle thereof), or a group of the formula:
-C(=O)NR⁴R⁵
(R⁴ and R⁵ are the same as defined above)), or an acid addition salt thereof.

2. The naphthyridine derivative according to claim 1, wherein Y is a phenyl group substituted by a group of the formula: D¹-E-F (D¹ and E are the same as defined in claim 2, and F is a group of the formula: 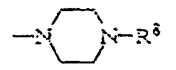 (R⁸ is the same as defined in claim 1), or an acid addition salt thereof.

3. The naphthyridine derivative according to claim 1, wherein D¹ is an oxygen atom, or an acid addition salt thereof.

4. The naphthyridine derivative according to claim 3, wherein F is a hydroxy group, a heteroaryl group or a group of the formula: -NR^{4'}R⁵ (R⁴ and R⁵ are the same as defined in claim 1), or an acid addition salt thereof.

5. The naphthyridine derivative according to any one of claims 1, 2, 3, or 4 wherein Ring A is an unsubstituted pyridine ring, or an acid addition salt thereof.

6. A medicament containing a naphthyridine derivative as set forth in any one of claims 1 to 5, or an acid addition salt thereof.

7. An acyl-CoA: cholesterol acyl transferase (ACAT) inhibitor which contains as an active ingredient a naphthyridine derivative as set forth in any one of claims 1 to 5, or an acid addition salt thereof.

8. A use of a naphthyridine derivative as set forth in any one of claims 1 to 5, or an acid addition salt thereof in the preparation of a pharmaceutical composition for treatment of hyperlipidemia or atherosclerosis.

## Revendications

1. Dérivé de naphtyridine de formule 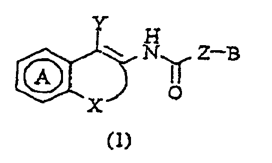 où le cycle A est un cycle pyridine substitué ou non-substitué choisi parmi les formules (a), (b) et (c) suivantes : 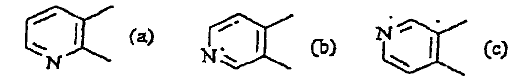 où le substituant du cycle pyridine est choisi dans le groupe constitué par un groupe alkyle en C₁-C₆, un atome d'halogène, un groupe cyano, un groupe trifluorométhyle, un groupe nitro, un groupe amino, un groupe mono(alkyle en C₁-C₆)amino, un groupe di(alkyle en C₁-C₆)amino, un groupe hydroxy, un groupe alcoxy en C₁-C₆, un groupe alkylthio en C₁-C₆, un groupe alkylsulfinyle en C₁-C₆ et un groupe alkylsulfonyle en C₁-C₆,
X est un groupe de formule : 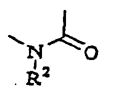 où R² est un groupe alkyle, un groupe alkyle substitué, un groupe alcényle ou un groupe alcényle substitué,
Z est un groupe : -NH-,
B est un groupe aromatique ou un groupe aromatique substitué,
et
Y est un groupe phényle, un groupe phényle substitué par un groupe alcoxy en C₁-C₆ ou un groupe phényle substitué par un groupe de formule : -D¹-E-F (D¹ est une liaison directe, un atome d'oxygène, un atome de soufre ou un groupe de formule : -NR³- (R³ est un atome d'hydrogène ou un groupe alkyle en C₁-C₆), E est un groupe hydrocarboné divalent ayant 1 à 6 atomes de carbone et contenant de façon optionnelle une liaison insaturée ou un groupe phénylène, F est un groupe hydroxy, un groupe carboxyle, un groupe (alcoxy en C₁-C₆)carbonyle, un groupe benzyloxycarbonyle, un atome d'halogène, un groupe cyano, un groupe benzyloxy, un groupe alcoxy en C₁-C₆, un groupe alcanoyloxy en C₁-C₆, un groupe alkylthio en C₁-C₆, un groupe alkylsulfinyle en C₁-C₆, un groupe alkylsulfonyle en C₁-C₆, un groupe alcanoylamino en C₁-C₆, un groupe alkylsulfonamido en C₁-C₆, un groupe phtalimido, un groupe hétéroaryle, un groupe de formule : -NR⁴R⁵ (R⁴ et R⁵ sont, de façon indépendante, un atome d'hydrogène ou un groupe alkyle en C₁-C₆ ou bien R⁴ et R⁵ peuvent, combinés avec l'atome d'azote adjacent auquel ils sont liés, former un groupe amino cyclique penta- à heptagonal saturé ayant de façon optionnelle un -NR⁸- (R⁸ est un atome d'hydrogène, un groupe alkyle en C₁-C₆, un groupe phényle ou un groupe benzyle) ou un atome d'oxygène dans son cycle), ou un groupe de formule :
-C(=O)NR⁴R⁵
(R⁴ et R⁵ sont tels que définis ci-dessus))
ou sel d'addition d'acide de ce dérivé.

2. Dérivé de naphtyridine selon la revendication 1, dans lequel Y est un groupe phényle substitué par un groupe de formule : D¹-E-F (D¹ et E sont tels que définis dans la revendication 1, et F est un groupe de formule : 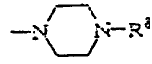 (R⁸ est tel que défini dans la revendication 1)),
ou sel d'addition d'acide de ce dérivé.

3. Dérivé de naphtyridine selon la revendication 1, dans lequel D¹ est un atome d'oxygène, ou sel d'addition d'acide de ce dérivé.

4. Dérivé de naphtyridine selon la revendication 1, dans lequel F est un groupe hydroxyle, un groupe hétéroaryle ou un groupe de formule : -NR⁴R⁵ (R⁴ et R⁵ sont tels que définis dans la revendication 1)),
ou sel d'addition d'acide de ce dérivé.

5. Dérivé de naphtyridine selon l'une quelconque des revendications 1, 2, 3 ou 4, dans lequel le cycle A est un cycle pyridine non-substitué,
ou sel d'addition d'acide de ce dérivé.

6. Médicament contenant un dérivé de naphtyridine selon l'une quelconque des revendications 1 à 5, ou un sel d'addition d'acide de ce dérivé.

7. Inhibiteur de l'acyl-CoA:cholestérol acyl transférase (ACAT) qui contient comme principe actif un dérivé de naphtyridine selon l'une quelconque des revendications 1 à 5, ou un sel d'addition d'acide de ce dérivé.

8. Utilisation d'un dérivé de naphtyridine selon l'une quelconque des revendications 1 à 5, ou d'un sel d'addition d'acide de ce dérivé dans la préparation d'une composition pharmaceutique pour le traitement de l'hyperlipidémie ou de l'athérosclérose.

## Patentansprüche

1. Naphthyridin-Derivat der Formel: 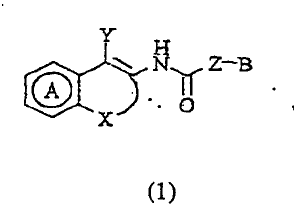 wobei Ring A ein substituierter oder unsubstituierter Pyridinring ausgewählt aus folgenden Formeln (a), (b) und (c) ist: 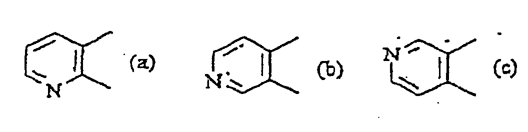 wobei der Substituent des Pyridinrings ausgewählt ist aus einem C₁-C₆-Alkylrest, einem Halogenatom, einer Cyanogruppe, einer Triflourmethylgruppe, einer Nitrogruppe, einer Aminogruppe, einem mono-C₁-C₆-Alkylaminorest, einem di-C₁-C₆-Alkylaminorest, einer Hydroxygruppe, einem C₁-C₆-Alkoxyrest, einem C₁-C₆-Alkylthiorest, einem C₁-C₆-Alkylsulfinylrest und einem C₁-C₆-Alkylsulfonylrest,
X ein Rest der Formel ist: 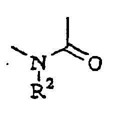 wobei R² ein Alkylrest, ein substituierter Alkylrest, ein Alkenylrest oder ein substituierter Alkenylrest ist,
Z ein Rest -NH- ist
B ein aromatischer Rest oder ein substituierter aromatischer Rest ist und Y eine Phenylgruppe, eine Phenylgruppe substituiert mit einem C₁-C₆-Alkoxyrest ist oder eine Phenylgruppe substituiert mit einem Rest der Formel:
-D¹-E-F
(D¹ ist eine direkte Bindung, ein Sauerstoffatom, ein Schwefelatom oder ein Rest der Formel: -NR³- (der Rest R³ ist ein Wasserstoffatom oder ein C₁-C₆-Alkylrest), E ist zweiwertiger Kohlenwasserstoffrest, der 1 bis 6 Kohlenstoffatome hat und gegebenenfalls eine ungesättigte Bindung oder einen Phenylenrest enthält, F ist eine Hydroxygruppe, eine Carboxylgruppe, ein C₁-C₆-Alkoxycarbonylrest, ein Benzyloxycarbonylrest, ein Halogenatom, eine Cyanogruppe, ein Benzyloxyrest, ein C₁-C₆-Alkoxyrest, ein C₁-C₆-Alkanoyloxyrest, ein C₁-C₆-Alkylthiorest, ein C₁-C₆-Alkylsulfinylrest, ein C₁-C₆-Alkylsulfonylrest, ein C₁-C₆-Alkanoylaminorest, ein C₁-C₆-Alkylsulfonamidorest, ein Phthalimidorest, ein Heteroarylrest, ein Rest der Formel: -NR⁴R⁵ (die Reste R⁴ und R⁵ sind unabhängig ein Wasserstoffatom oder ein C₁-C₆-Alkylrest, oder die Reste R⁴ und R⁵ können sich mit dem benachbarten Stickstoffatom, zu dem sie gebunden sind, verbinden, um einen gesättigten 5- bis 7-gliedrigen cyclischen Aminorest, der gegebenenfalls einen Rest -NR⁸- besitzt (der Rest R⁸ ist ein Wasserstoffatom, ein C₁-C₆-Alkylrest, ein Phenylrest oder ein Benzylrest) zu bilden, oder ein Sauerstoffatom in dem Ring davon), oder ein Rest der Formel: -C(=O)NR⁴R⁵ (R⁴ und R⁵ haben die gleiche Bedeutung wie vorstehend)), oder ein Säureadditionssalz davon.

2. Naphthyridin-Derivat gemäß Anspruch 1, wobei Y ein Phenylrest ist, substituiert mit einem Rest der Formel: -D¹-E-F (D¹ und E haben die gleiche Bedeutung wie in Anspruch 1 und F ist ein Rest der Formel: 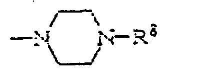 (R⁸ hat die gleiche Bedeutung wie in Anspruch 1)), oder ein Säureadditionssalz davon.

3. Naphthyridin-Derivat gemäß Anspruch 1, wobei D¹ ein Sauerstoffatom ist oder ein Säureadditionssalz davon.

4. Naphthyridin-Derivat gemäß Anspruch 1, wobei F eine Hydroxygruppe, ein Heteroarylrest oder ein Rest der Formel: -NR⁴R⁵ ist (R⁴ und R⁵ haben die gleiche Bedeutung wie in Anspruch 1), oder ein Säureadditionssalz davon.

5. Naphthyridin-Derivat gemäß einem der Ansprüche 1, 2, 3 oder 4, wobei Ring A ein unsubstituierter Pyridinring ist oder ein Säureadditionssalz davon.

6. Medikament, das ein Naphthyridin-Derivat, wie in einem der Ansprüche 1 bis 5 dargelegt, oder ein Säureadditionssalz davon enthält.

7. Acyl-CoA: Cholesterinacyltransferase (ACAT)-Inhibitor, der als Wirkstoff ein Naphthyridin-Derivat wie in einem der Ansprüche 1 bis 5 dargelegt, oder ein Säureadditionssalz davon enthält.

8. Verwendung eines Naphthyridin-Derivats wie in einem der Ansprüche 1 bis 5 dargelegt oder eines Säureadditionssalz davon für die Herstellung eines Arzneimittels zur Behandlung von Hyperlipidämie und Atherosklerose.
